# FASCICULE DE BREVET EUROPEEN

(11) **EP 1 461 342 B1**
(45) Date de publication et mention de la délivrance du brevet: **07.03.2007**
(21) Numéro de dépôt: 02805806.3
(22) Date de dépôt: 23.12.2002
(51) Int. Cl.: C07D 498/18, A61K 31/4188, A61P 31/04

(54) **DERIVES DE STREPTOGRAMINES,LEUR PREPARATION ET LES COMPOSITIONS QUI LES CONTIENNENT**
STREPTOGRAMINDERIVATE, DEREN HERSTELLUNG UND ZUSAMMENSETZUNGEN, DIE DIESE ENTHALTEN
STREPTOGRAMIN DERIVATIVES, PREPARATION THEREOF AND COMPOSITIONS CONTAINING SAME

(30) Priorité: 26.12.2001 FR 0116856
(43) Date de publication de la demande: 29.09.2004
(73) Titulaire: Aventis Pharma S.A., 92160 Antony (FR)
(72) Inventeur: BACQUE, Eric, F-91190 Gif sur Yvette (FR); BARRIERE, Jean-Claude, deceased (FR); PUCHAULT, Gérard, F-77139 Marcilly (FR)
(86) Numéro de dépôt international: PCT/FR2002/004529
(87) Numéro de publication internationale: WO 2003/055891

(56) Documents cités:
- GB-A- 2 206 879
- US-A- 5 242 938

## Description

La présente invention concerne des dérivés du groupe A des streptogramines de formule générale : 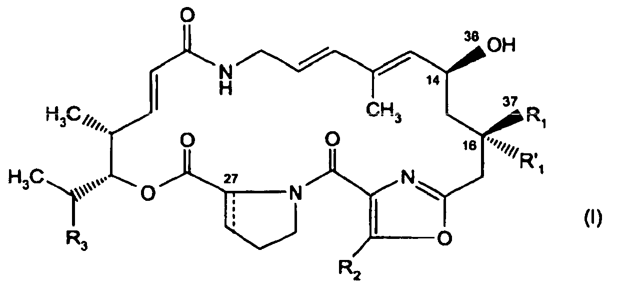 qui présentent une activité antibactérienne particulièrement intéressante.

Parmi les streptogramines connues, la pristinamycine (RP 7293), antibactérien d'origine naturelle produit par *Streptomyces pristinaespiralis* a été isolée pour la première fois en 1955. La pristinamycine commercialisée sous le nom de Pyostacine^{®} est constituée principalement de pristinamycine IIA associée à la pristinamycine IA.

Un autre antibactérien de la classe des streptogramines : la virginiamycine, a été isolé à partir de *Streptomyces virginiae,* ATCC 13161 [Antibiotics and Chemotherapy, 5, 632 (1955)]. La virginiamycine (Staphylomycine^{®}) est constituée principalement de facteur M₁ (VM1) associé au facteur S (VS).

Le brevet US-A-5 242 938 se rapporte à des Virginiamycines M1 qui sont préparés par des substitutions en position 14-OH ou /et 16-OH.

F. Le Goffic et coll., Eur J. Med. Chem. Chimica Therapeutica, 16(1), 69-72 (1981) ont décrit la préparation de dérivés dihydroxylés de la pristinamycine IIA.

Dans la demande de brevet GB 2 206 879 ont été décrits des dérivés modifiés du groupe A des streptogramines de structure : 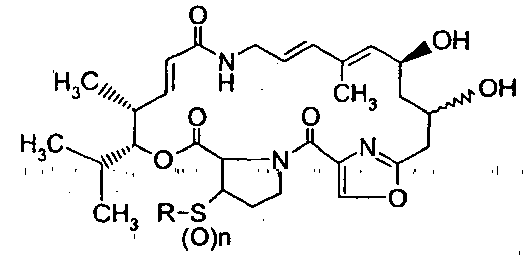 cependant ces dérivés ne manifestaient qu'une faible activité.

Il a été maintenant trouvé, que les dérivés de streptogramine du groupe A de formule générale (I) dans laquelle :
■ R₁ représente un atome d'halogène ou un radical hydroxy, alcoyloxy, azido, thiocyanato, ou R₁ représente un radical -NR'R" pour lequel R' est un atome d'hydrogène ou un radical méthyle, et R" est un atome d'hydrogène, un radical alcoyle, cycloalcoyle, allyle, propynyle, benzyle, ou -OR'", R"' étant un atome d'hydrogène, un radical alcoyle, cycloalcoyle, allyle, propynyle ou benzyle, ou bien R" représente -NR°R°°, R° et R°° pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, et
■ R'₁ représente un atome d'hydrogène ou bien,
■ R₁ et R'₁ forment ensemble un radical oxo,
■ R₂ représente un atome d'halogène ou un radical alcoyle, alcènyle ou alcynyle, phényle ou hétéroaryle, ou un radical alcoylthio, phénylthio ou hétéroarylthio,
■ R₃ est un atome d'hydrogène ou un radical méthyle ou éthyle, et
■ la liaison 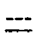 représente une liaison simple (stéréochimie 27R) ou une liaison double,
les radicaux alcoyle contenant 1 à 6 carbones en chaîne droite ou ramifiée et les radicaux alcényle et/ou alcynyle contenant 2 à 6 carbones en chaîne droite ou ramifiée, présentent une activité antibactérienne particulièrement intéressante notamment sur des germes bactériens résistants, seuls ou associés à un dérivé de streptogramine du groupe B.

Selon l'invention, lorsque R₁ et ou R₂ est un atome d'halogène, il peut représenter le fluor, le chlore, le brome ou l'iode ;
lorsque R₂ est un radical hétéroaryle ou hétéroarylthio, ce dernier est mono ou bicyclique et comprend 1 à 4 hétéroatomes choisis parmi l'azote, l'oxygène ou le soufre.
A titre d'exemple, lorsque R₂ est un radical hétéroaryle ou hétéroarylthio, le radical hétéroaryle peut être choisi parmi pyridyle, thiényle, furyle, pyrrolyle, imidazolyle, pirazinyle, quinolyle, isoquinolyle, indolyle, oxazolyle, thiazolyle, pyrazolyle, indazolyle, thiadiazolyle ou oxadiazolyle.

Selon l'invention, les dérivés de streptogramine de formule générale (I) peuvent être préparés par métallation d'un dérivé de streptogramine de formule générale : 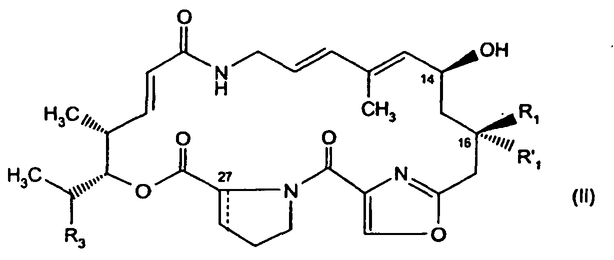 dans laquelle R'₁, R₃ et la liaison 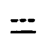 sont définis comme précédemment et R₁ représente un atome de fluor ou un radical hydroxy, alcoyloxy, ou représente un radical -NR'R" pour lequel R' est H ou un radical méthyle, et R" est H, un radical alcoyle, cycloalcoyle, allyle, propynyle, benzyle, ou -OR"', R"' étant H, alcoyle, cycloalcoyle, allyle, propynyle ou benzyle, ou bien R" représente -NR°R°°, R° et R°° pouvant être méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, ou bien, R₁ et R'₁ forment ensemble un radical oxo,
par action successivement d'un amidure pour donner un polyanion répondant, selon la nature des substituants et selon que l'on opère à partir d'un dérivé de streptogramines de formule générale (II) pour lequel la liaison 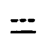 est une liaison double ou une liaison simple, aux structures de formules générales : 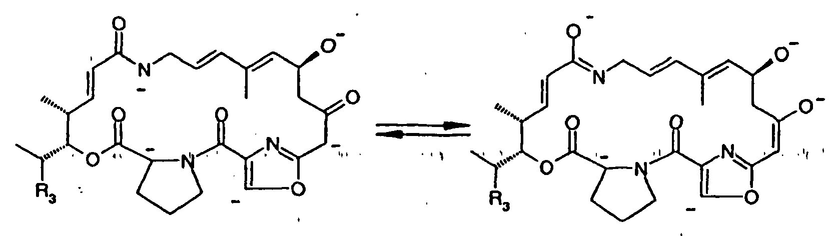 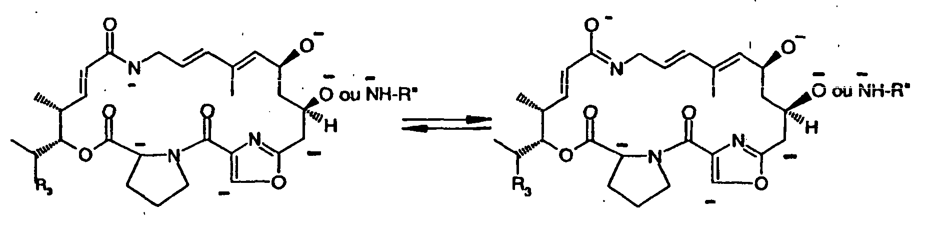 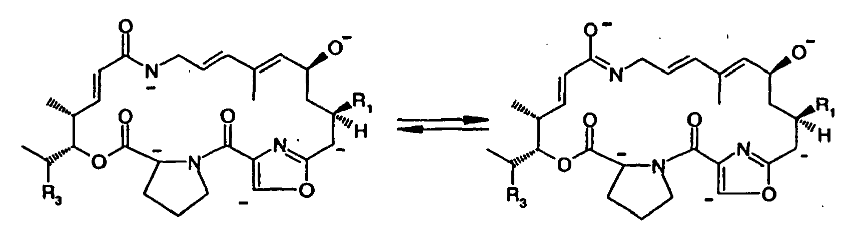 ou 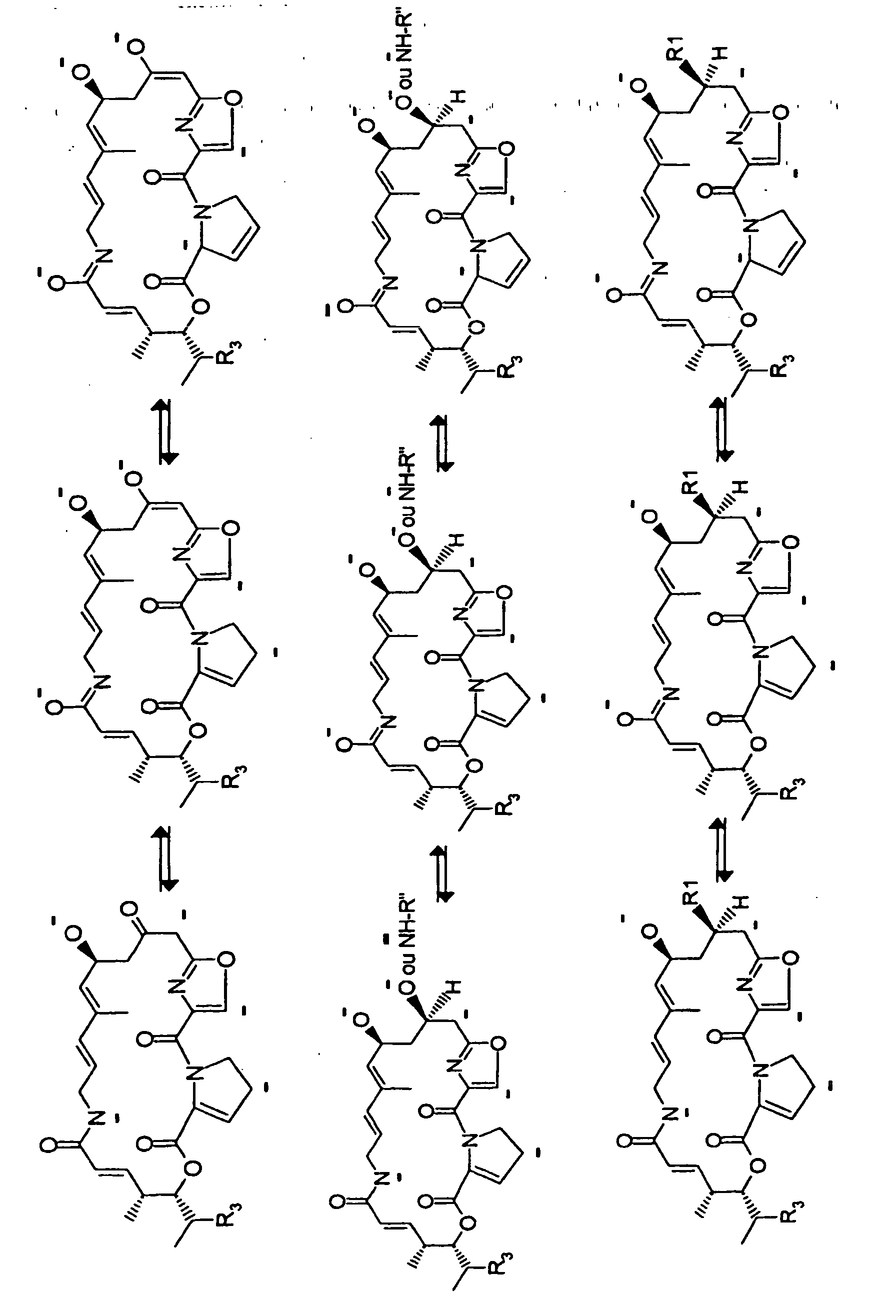 et pour lesquelles R₃ est défini comme précédemment, et R₁ représente un atome de fluor ou un radical alcoyloxy, ou représente un radical -NR'R" pour lequel R' est un radical méthyle, et R" est un radical alcoyle, cycloalcoyle, allyle, propynyle, benzyle, ou -OR''',R'" étant H, alcoyle, cycloalcoyle, allyle, propynyle ou benzyle, ou bien R" représente -NR°R°°, R° et R°° pouvant être méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, puis par action d'un agent électrophile pouvant être représenté par la formule générale :

R₂-X (III)

dans laquelle R₂ est un atome d'halogène, un radical alcoyle, un radical alcènyle ou alcynyle, à l'exception de représenter un radical vinyle ou éthynyle, et X représente un atome d'halogène ou un radical sulfonyloxy, ou bien par action d'un disulfure lorsque R₂ est alcoylthio, phénylthio ou hétéroarylthio, suivie le cas échéant de la transformation d'un dérivé de streptogramine de formule générale (I) pour lequel R₂ est un atome de chlore, brome ou iode, en un dérivé de streptogramine de formule générale (I) pour lequel R₂ est radical vinyle, éthynyle, phényle ou hétéroaryle et/ou le cas échéant, transforme le dérivé de streptogramine de formule générale (I) pour lequel R₁ et R'₁ forment ensemble un radical oxo en un dérivé correspondant pour lequel R₁ est un atome de chlore, de brome ou d'iode ou un radical azido ou thiocyanato et R'₁ est un atome d'hydrogène et/ou suivie de la transformation d'un dérivé de streptogramine de formule générale (I) pour lequel la liaison 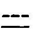 est une liaison double en un dérivé de streptogramine de formule générale (I) pour lequel la liaison 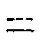 est une liaison simple.

La réaction de métallation s'effectue généralement à une température comprise entre -100 et -20°C, en présence de 4 à 7 équivalents d'un amidure, de préférence un amidure de lithium, par exemple choisi parmi le diisopropylamidure de lithium ou le 2,2,6,6-tétraméthylpipéridine amidure de lithium, dans un solvant anhydre notamment un éther comme par exemple le tétrahydrofuranne anhydre, éventuellement en présence d'un co-solvant comme la 1,3-diméthyl-3,4,5,6-tétrahydro-2(1H)-pyrimidinone ou l'hexaméthylphosphorotriamide, en présence ou non d'additifs comme par exemple la tétraméthyléthylène diamine, le diazabicyclo[2,2,2]octane ou le chlorure de lithium anhydre. Il est avantageux d'opérer en milieu inerte par exemple sous argon.

La réaction de l'agent électrophile [notamment représenté par la formule générale (III)] s'effectue à une température comprise entre -80 et 20°C. Le réactif électrophile est avantageusement choisi parmi les halogénures ou les sulfonates d'alcoyle, d'allyle, de propargyle, de benzyle ou d'hétéroarylméthyle, ou encore choisi parmi le brome ou l'iode ou le chlorure d'iode, il peut également être choisi parmi les donneurs d'halogène (N-bromo ou N-chloro succinimide, 1,1,2,2-tétra-fluoro-1,2-dichloro ou dibromo éthane, le 1,2-dibromo-1,1,2,2-tétrachloro éthane, le 1,2-diiodoéthane ou le 1,2-dibromoéthane, le 1-chloro-2-iodo éthane, l'hexachloroacétone, par exemple). On opère avantageusement dans un solvant organique comme un éther, tétrahydrofurane par exemple.

L'opération subséquente de transformation d'un dérivé de streptogramine de formule générale (I) pour lequel R₂ est chlore, brome ou iode, en un dérivé de streptogramine de formule générale (I) pour lequel R₂ est vinyle, éthynyle, phényle ou hétéroaryle s'effectue de préférence à partir d'un dérivé pour lequel l'halogène est brome ou iode, par action d'un dérivé de l'étain (vinyl tributyl étain, vinyl triméthyl étain par exemple), ou d'un dérivé du bore (acide boronique, ou dérivé du 9-bora-bicyclo[3,3,1]nonane par exemple), ou d'un dérivé du zinc (par exemple de structure R₂ZnCl) en présence d'un catalyseur comme un dérivé du palladium (par exemple le tétrakis-triphénylphosphine palladium) en présence ou non d'une phosphine (comme par exemple la tétrakis-triphénylphosphine) d'une base (comme par exemple une base azotée : triéthylamine, diéthylamine, pipéridine ou un carbonate : carbonate de sodium ou de potassium) et éventuellement en présence d'un co-catalyseur comme un sel de cuivre (CuI), dans un solvant organique inerte, ou un mélange de solvants comme un hydrocarbure aromatique (le toluène par exemple), un nitrile (acétonitrile), un amide (comme par exemple le diméthylformamide) ou un éther (tétrahydrofuranne) à une température comprise entre 20°C et la température de reflux du mélange réactionnel.

L'opération subséquente de transformation d'un dérivé de streptogramine de formule générale (I) pour lequel R₁ et R'₁ forment ensemble un radical oxo en un dérivé correspondant pour lequel R₁ est un atome de chlore, de brome ou d'iode ou un radical azido ou thiocyanato et R'₁ est un atome d'hydrogène s'effectue selon la demande décrite dans la demande de brevet international WO O1/02427 incorporée ici à-titre de référence.

Lorsque R₁ et R'₁ sont définis comme précédemment à l'exception de représenter ensemble un radical oxo, l'opération subséquente de transformation d'un dérivé de streptogramine de formule générale (I) pour lequel la liaison 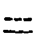 est une liaison double en un dérivé de streptogramine de formule générale (I) pour lequel la liaison 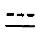 est une liaison simple (stéréochimie 27R), s'effectue généralement par réduction au moyen d'un hydrure alcalin (borohydrure de sodium, borohydrure de lithium par exemple), dans un solvant tel qu'un éther (tétrahydrofuranne par exemple), à une température comprise entre 0 et 60°C. La séparation des 2 épimères en C₂₇ s'effectue selon les méthodes habituelles qui n'altèrent pas le reste de la molécule, notamment par cristallisation ou chromatographie.

Les dérivés de streptogramine du groupe A de formule générale (II) pour lesquels R₁ est un atome de fluor ou un radical -NR'R" pour lequel R' est un atome d'hydrogène ou un radical méthyle, et R" est un atome d'hydrogène, un radical alcoyle, cycloalcoyle, allyle, propynyle, benzyle, ou -OR"', R"' étant un atome d'hydrogène, un radical alcoyle, cycloalcoyle, allyle, propynyle ou benzyle, ou bien R" représente -NR°R°°, R° et R°° pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, peuvent être préparés selon ou par analogie avec les méthodes décrites dans les demandes internationales WO 99/05165 et WO 01/02427.

Le dérivé dihydroxylé de streptogramine de formule générale (II) peut être obtenu selon les méthodes décrites par F. Le Goffic et coll., Eur J. Med. Chem. Chimica Therapeutica, 16(1), 69-72 (1981) ou dans la demande de brevet FR 2 795 733.

La séparation de la forme épimère 16R et de la forme épimère 16S s'effectue selon les méthodes habituelles ; par exemple la séparation des formes épimères peut s'effectuer par chromatographie, chromatographie flash, chromatographie liquide haute performance (CLHP), sur phase chirale ou non, ou chromatographie par partage centrifuge (CPC), à partir du mélange des épimères 16R et 16S ou par cristallisation.

Les dérivés de streptogramine de formule générale (II) pour lesquels R₁ est un radical alcoyloxy peuvent être préparés par action d'un dérivé de formule générale :

alk-X (V)

pour lequel alk représente le radical alcoyle correspondant et X représente un atome d'halogène, ou un radical méthylsulfonyloxy, p.toluènesulfonyloxy ou trifluorométhylsulfonyloxy, en présence d'un agent de transfert de phase, sur un dérivé dihydroxylé de streptogramine de formule générale : 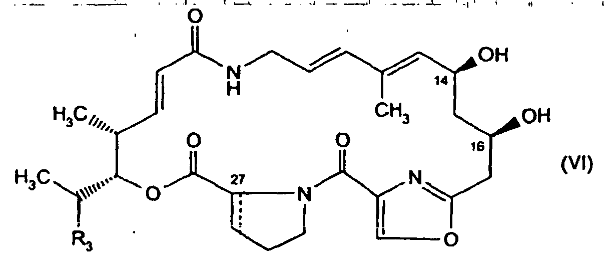 dans laquelle R₃ et la liaison 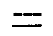 sont définis comme précédemment, et dont éventuellement la fonction hydroxy en position 14 et/ou la fonction amide en position 8 ont été préalablement protégées, suivie le cas échéant de l'élimination du/des radical(aux) protecteur(s).

De préférence, lorsque X est un atome d'halogène, on fait agir un dérivé de formule générale (V) pour lequel X est un atome de brome ou d'iode.

L'agent de transfert de phase est avantageusement choisi parmi les dérivés d'ammonium quaternaire (par exemple les sels de tétraalcoylammonium ou de trialcoylbenzylammonium comme le chlorure, le bromure ou le sulfate)

La réaction s'effectue généralement en milieu basique, par exemple en présence d'hydroxyde de sodium ou de potassium, ou en présence de carbonate de potassium ou de carbonate de césium, à une température comprise entre 10 et 60°C, en milieu hydro-organique, par exemple dans un hydrocarbure (toluène par exemple), un solvant halogéné (par exemple dichlorométhane) ou un ester (acétate d'éthyle notamment). De préférence on opère à environ 20°C. De préférence également on opère en présence d'un excès du dérivé de formule générale (V).

La protection et la déprotection du radical hydroxy en position 14 ou de l'amide en position 8 s'effectuent selon les méthodes habituelles qui n'affectent pas le reste de la molécule, notamment par application des méthodes décrites par T.W. Greene et P.G.M. Wuts, Protective Groups in Organic Synthesis (2^{ème} éd.), A. Wiley - Interscience Publication (1991), ou par Mc Omie, Protective Groups in Organic Chemistry, Plenum Press (1973). Par exemple la protection du radical hydroxy s'effectue par un radical allyle qui est mis en place et éliminé selon les méthodes connues. La protection de l'amide peut s'effectuer notamment par le radical -t:butoxyearbonyle. ,

Lorsque la réaction conduit à un mélange des isomères 14- et 16-O-alcoylés, ces derniers peuvent être séparés selon les méthodes habituelles qui n'altèrent, pas le reste de la molécule, notamment par chromatographie [Chromatographie Liquide Haute Performance (CLHP) sur phase normale ou inverse, sur phase chirale ou non ou par chromatographie flash] ou par cristallisation.

Les dérivés de pristinamycine de formule générale (IV) correspondent respectivement à la pristinamycine IIA (PIIA), à la pristinamycine IIB (PIIB), à la pristinamycine IIC (PIIC), à la pristinamycine IID (PIID), à la pristinamycine IIF (PIIF) et à la pristinamycine IIG (PIIG) qui sont des composantes connues de la pristinamycine naturelle. Les composantes PIIF et PIIG ont été décrites dans le brevet européen EP 614910. La pristinamycine IIC (PIIC), et la pristinamycine IID (PIID) peuvent être obtenues comme décrit par J.C. Barrière et coll., Expert. Opin. Invest. Drugs, 3(2), 115-31 (1994).

Les dérivés de pristinamycine de formule générale (IV) sont représentés ci-dessous: 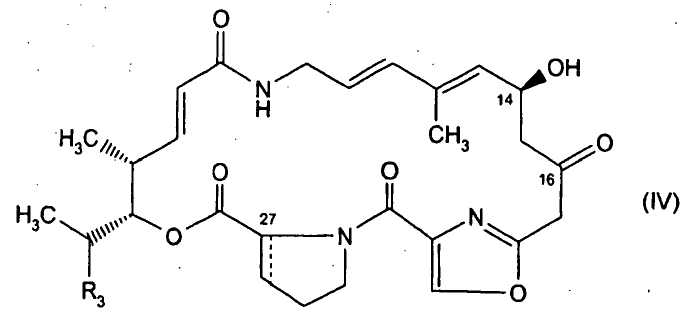 dans laquelle
R₃ est un atome d'hydrogène ou un radical méthyle ou éthyle, et
la liaison 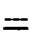 représente une liaison simple (stéréochimie 27R) ou une liaison double.

La préparation et la séparation des composantes des streptogramines naturelles du groupe A [streptogramines de formule générale (IV)] est effectuée par fermentation et isolement des constituants à partir du moût de fermentation selon ou par analogie avec la méthode décrite par J. Preud'homme et coll., Bull. Soc. Chim. Fr., vol. 2, 585 (1968) ou dans le brevet européen EP 614910. Alternativement la préparation des composantes naturelles du groupe A peut être effectuée par fermentation spécifique, comme décrit dans la demande de brevet FR 2 689 518.

Les dérivés de streptogramine de formule générale (I) peuvent être purifiés le cas échéant par des méthodes physiques telles que la cristallisation, la chromatographie ou la CPC.

Les dérivés de streptogramine de formule générale (I) Pour lesquels R₁ représente un radical -NR'R" peuvent être transformés à l'état de sels d'addition avec les acides, par les méthodes connues. Il est entendu que ces sels, lorsque'ils existent, entrent aussi dans le cadre de la présente invention.
Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, éthanesulfonates, phénylsulfonates, p.toluènesulfonates, iséthionates, naphtylsulfonates ou camphorsulfonates, ou avec des dérivés de substitution de ces composés).

Il est entendu que les associations formées au moyen des dérivés selon l'invention et des streptogramines du groupe B entrent également dans le cadre de la présente invention.

Les dérivés de streptogramine selon la présente invention présentent des propriétés antibactériennes et des propriétés synergisantes de l'activité antibactérienne des dérivés de streptogramine du groupe B. Ils sont particulièrement intéressants du fait de leur activité puissante seuls ou associés.

Lorsqu'ils sont associés avec une composante ou un dérivé du groupe B des streptogramines, ces derniers peuvent être choisis selon que l'on désire obtenir une forme administrable par voie orale ou parentérale, parmi les composantes naturelles : pristinamycine IA, pristinamycine IB, pristinamycine IC, pristinamycine ID, pristinamycine IE, pristinamycine IF, pristinamycine IG, virginiamycine S1, S3 ou S4, vernamycine B ou C, étamycine ou parmi des dérivés d'hémisynthèse tels que décrits dans les brevets ou demandes de brevet US 4618599, US 4798827, US 5326782, US 5786449, WO 01/10895, WO 01/07467, EP 772630, EP 770132 ou EP 1056071.

Les dérivés de streptogramine selon la présente invention présentent des propriétés antibactériennes et des propriétés synergisantes de l'activité antibactérienne des dérivés de streptogramine du groupe B, notamment sur *Staphylococcus Epidermidis* N52 ou sur souches résistantes comme *Staphylococcus aureus* Stephan. Ils sont particulièrement intéressants du fait de leur activité puissante seuls ou associés.

In vitro sur *Staphylococcus Epidermidis* N52, les dérivés de streptogramine selon l'invention se sont montrés actifs à des concentrations comprises entre 1 et 64 µg/ml seuls ou entre 0,25 et 32 µg/ml associés à un dérivé du groupe B comme la pristinamycine IB. In vitro sur *Staphylococcus aureus* Stephan ils se sont montrés actifs à des concentrations comprises entre 0,5 et 64 µg/ml.
Enfin, les produits selon l'invention n'ont pas révélé de toxicité.

Les exemples suivants, donnés à titre non limitatif, illustrent la présente invention.

Dans les exemples qui suivent, la nomenclature 16-désoxo pristinamycine IIA (ou IIB) signifie le remplacement de la fonction cétone en position 16 par 2 atomes d'hydrogène. Au fur et à mesure de la chromatographie, toutes les fractions sont analysées par chromatographie en couche mince (CCM), sur plaques de silice Merck 60F254. Les fractions correspondants à un même tâche en CCM sont regroupées puis concentrées à sec, sous pression réduite (30°C; 2,7 kPa). Les résidus ainsi obtenus sont analysés par les techniques spectroscopiques habituelles (RMN; IR; MS) ce qui permet d'identifier le produit attendu.

### Exemple 1

### (16R)-20-Bromo- 16-désoxo- 16-fluoro pristinamycine II_{B}

A 5,32 g de (16*R*)-16-désoxo-16-fluoro pristinamycine II_{B} en solution dans 100 cm³ de tétrahydrofuranne anhydre, on additionne, sur une heure, à -72°C, sous atmosphère d'argon, une solution de diisopropyl amidure de lithium, refroidie à -50°C. Cette dernière a été préalablement préparée par addition, à -50°C, de 31 cm³ de n-butyl lithium (1,6 M dans l'hexane) à une solution de 100 cm³ de tétrahydrofuranne et de 7 cm³ de diisopropyl amine suivie d'une remontée à 20°C après l'addition. A la solution brune et épaisse résultante maintenue à -72°C, est ajoutée rapidement 7,5 cm³ de tétraméthyl éthylène diamine. Après agitation du mélange réactionnel dix minutes à -72°C, on additionne toujours à -72°C, en quinze minutes, 9,8 g de 1,2-dibromo 1,1,2,2-tétrachloro éthane en solution dans 20 cm³ de tétrahydrofuranne. Le mélange est agité 1 heure à -72°C avant addition, goutte à goutte, de 10 cm³ d'acide acétique puis de 100 cm³ d'eau. La solution obtenue revenue à 20°C est concentrée sous pression réduite. Le résidu est repris par 100 cm³ d'eau et 100 cm³ d'acétate d'éthyle. La phase organique est séparée et la phase aqueuse extraite par 100 cm³ d'acétate d'éthyle. Les phases organiques rassemblées sont lavées par 50 cm³ d'eau additionnés d'acide chlorhydrique 1N de façon à ajuster le pH de la phase aqueuse à 2. La phase organique résultante est séparée, successivement lavée par 50 cm³ d'eau, par une solution aqueuse de bicarbonate de sodium à 5 % et par 50 cm³ de saumure puis séchée sur sulfate de sodium, filtrée et concentrée à sec sous pression réduite (2,7 kPa) pour donner 10,3 g d'une meringue orange. Ce résidu est purifié par deux chromatographies flash successives sur silice [éluant : Acétate d'éthyle / Cyclohexane (70/30 en volumes) puis dichlorométhane / méthanol (97/3 en volumes)]. Après concentration sous pression réduite (2,7 kPa) des fractions contenant l'attendu, on obtient 0,53 g de (16*R*)-20-bromo-16-désoxo-16-fluoro pristinamycine II_{B}, sous forme d'une poudre jaune pâle fondant vers 172°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,12 (d, J = 6,5 Hz : 3H) ; de 1,55 à 2,15 (mt : 5H) ; 1,81 (s : 3H) ; 2,16 (mt : 1H) ; 2,27 (mt : 1H) ; 2,77 (mt : 1H) ; 2,91 (ddd, J = 26 - 17 et 5 Hz : 1H) ; 3,18 (t dédoublé, J= 17 et 7 Hz : 1H) ; 3,48 (mt : 1H) ; 3,65 (mt : 1H) ; 4,06 (mt : 1H) ; 4,58 (mt : 1H) ; 4,81 (dd, J = 10 et 1,5 Hz : 1H) ; de 4,80 à 4,90 (mt : 1H); 4,87 (dd, J = 9 et 4 Hz : 1H) ; 5,12 (dmt, J_{HF} = 48 Hz : 1H) ; 5,43 (d large, J = 9 Hz : 1H) ; 5,71 (ddd, J = 16 - 8,5 et 4 Hz : 1H) ; 5;83 (dd, J = 16 et 1,5 Hz : 1H) ; 5,94 (mt : 1H) ; 6,24 (d, J = 16 Hz : 1H) ; 6,53 (dd, J = 16 et 5 Hz : 1H).

### Exemple 2

### (16R)-16-Désoxo-16-fluoro-20-rnéthyl-pristinamycine II_{B}

A une solution refroidie à 0°C de 0,76 g de (16*R*)-16-désoxo-16-fluoro-20-méthyl pristinamycine II_{A} dans 20 cm³ de tétrahydrofuranne, on additionne rapidement 0,87 cm³ de borohydrure de lithium (2 M dans le tétrahydrofuranne). Après addition, le mélange réactionnel est agité à 0°C durant 3,5 heures avant ajout de 10 cm³ d'acide chlorhydrique 1N et de 10 cm³ d'eau. Le pH du mélange obtenu est ajusté à 6,7 par addition d'une solution aqueuse de bicarbonate de sodium. Le mélange est extrait par 2 fois 35 cm³ de dichlorométhane. Les phases organiques sont rassemblées puis lavées par 30 cm³ de saumure, séchées sur sulfate de sodium, filtrées et concentrées à sec sous pression réduite (2,7 kPa) pour donner 0,63 g d'une meringue blanche que l'on purifie par HPLC semi-préparative [phase Kromasil C8 10 µm; éluant : Eau /Acétonitrile (60 /40 en volumes)]. Les fractions contenant le produit attendu sont extraites par 2 fois 30 cm³ de dichlorométhane. Les phases organiques sont rassemblées puis séchées sur sulfate de sodium, filtrées et concentrées à sec sous pression réduite (2,7 kPa) pour donner 0,145 g d'un solide blanc. Celui-ci est agité 1 heure dans un mélange pentane / éther diisopropylique, filtré puis séché une nuit sous pression réduite (2,7 kPa) pour donner 0,12 g de (16*R*)-16-Désoxo-16-fluoro-20-méthyl-pristinamycine II_{B}, sous forme d'une poudre blanche fondant vers 137 °C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,11 (d, J = 6,5 Hz : 3H) ; 1,65 (d, J = 3,5 Hz : 1H) ;de 1,65 à 2,05 (mt : 5H) ; 1,81 (s : 3H) ; 2,13 (mt : 1H) 2,22 (mt : 1H) ; 2,53 (s : 3H) ; 2,76 (mt : 1H) ; 2,86 (ddd, J = 24 - 17 et 5 Hz : 1H) ; 3,12 (t dédoublé, J = 17 et 7 Hz : 1H) ; 3,48 (ddd, J = 16 - 9 et 3 Hz : 1H) ; 3,73 (mt : 1H) ; 4,08 (mt : 1H) ; 4,55 (ddd large, J = 16 - 9 et 3 Hz : 1H) ; 4,81 (dd, J = 10 et 2 Hz : 1H) ; de 4,80 à 4,90 (mt : 1H); 4,85 (dd, J = 9 et 3,5 Hz : 1H); 5,12 (dmt, J_{HF} = 48 Hz : 1H); 5,42 (d large, J = 9 Hz : 1H) ; 5,70 (ddd, J = 16 - 9 et 4 Hz : 1H) ; 5,82 (dd, J = 16 et 2 Hz : 1H) ; 5,94 (mt : 1H) ; 6,23 (d large, J = 16 Hz : 1 H) ; 6,52 (dd, J = 16 et 5 Hz : 1 H).

La (16*R*)-16-désoxo-16-fluoro-20-méthyl pristinamycine II_{A} peut-être obtenue de la façon suivante.

A 10,6 g de (16*R*)-16-désoxo-16-fluôro pristinamycine II_{A} dans 300 cm³ de tétrahydrofuranne anhydre et 20 cm³ de 1,3-diméthyl-3,4,5,6-tétrahydro-2(1H)-pyrimidinone, on additionne, en une heure, à -72°C, sous atmosphère d'argon, une solution de diisopropyl amidure de lithium, refroidie à -50°C. (Cette dernière a été préalablement préparée par addition, à -30°C, de 50 cm³ de n-butyl lithium (1,6 M dans l'hexane) à une solution de 100 cm³ de tétrahydrofuranne et de 11,2 cm³ de diisopropyl amine suivie d'une remontée à 20°C après l'addition). A la solution brune et épaisse résultante maintenue à -72°C, est ajoutée rapidement 12,1 cm³ de tétraméthyl éthylène diamine. Après agitation du mélange réactionnel dix minutes à -72°C, on additionne, toujours à -72°C, en cinq minutes, 12,4 g d'iodure de méthyle. Le mélange est agité 1 heure à -72°C avant addition, goutte à goutte, de 12,6 cm³ d'acide acétique puis de 60 cm³ d'eau. La solution obtenue revenue à 20°C est concentrée sous pression réduite. L'huile épaisse brune obtenue est repris par 200 cm³ d'eau et 500 cm³ d'acétate d'éthyle. On additionne de l'acide chlorhydrique concentré (10 cm³) de façon à ajuster le pH de la phase aqueuse à 3. La phase organique est séparée et la phase aqueuse extraite par 200 cm³ d'acétate d'éthyle. Les phases organiques rassemblées sont lavées par 200 cm³ d'eau additionnés de bicarbonate de sodium de façon à obtenir un pH final de 7-8. La phase organique résultante est séparée, lavée par 100 cm³ de saumure puis séchée sur sulfate de sodium, filtrée et concentrée à sec sous pression réduite (2,7 kPa) pour donner 15,1 g d'une huile brune que l'on purifie par deux chromatographies flash successives [éluant : Acétate d'éthyle / Cyclohexane (50/50 en volumes) puis dichlorométhane / méthanol /acétonitrile (94/3/3 en volumes)]. Après concentration sous pression réduite (2,7 kPa), on obtient 1 g de (16*R*)-16-désoxo-16-fluoro-20-méthyl pristinamycine II_{A}, sous forme d'une poudre blanche fondant vers 178°C.

Spectre de R.M.N. ¹H (400 MHz, CDCl₃, δ en ppm) : 0,99 (d, J = 6,5 Hz : 6H) ; 1,13 (d, J = 6,5 Hz : 3H) ; 1,52 (d, J = 4 Hz : 1H) ; 1,75 (s : 3H) ; de 1,95 à 2,20 (mt : 2H) ; 2,27 (mt : 1H) ; 2,41 (s : 3H) ; de 2,60 à 2,90 (mt : 3H) ; 2,97 (mt : 1H) ; 3,18 (t dédoublé, J = 14,5 et 3,5 Hz : 1H) ; 3,85 (d très large, J = 18 Hz : 1H) ; 4,08 (mt : 1H) ; 4,29 (mt : 2H) ; de 4,40 à 4,70 (mt : 1H) ; 4,62 (mt : 1H) ; 4,94 (mt : 2H) ; 5,66 (dt, J = 16 et 4 Hz : 1H) ; 5,91 (d large, J = 16 Hz : 1H) ; 5,99 (d large, J = 16 Hz : 1H) ; 6,14 (t, J = 3 Hz: 1H) ; 6,59 (dd, J =-16 et 7 Hz: 1H) ; 7,07 (mf: 1H).

### Exemple 3

### (16R)-16-Désoxo-16-diméthylamino-20-méthyl-pristinamycine II_{B}

A une solution de 1 g de (16*R*)-16-désoxo-16-diméthylamino pristinamycine II_{A} dans 40 cm³ de tétrahydrofuranne anhydre, on additionne goutte à goutte, à -75°C, sous atmosphère d'argon, une solution de diisopropyl amidure de lithium. Cette dernière a été préalablement préparée par addition, à -15°C, de 5,6 cm³ de n-butyl lithium (1,6 M dans l'hexane) à une solution de 10 cm³ de tétrahydrofuranne et de 1,26 cm³ de diisopropyl amine. La solution brune sombre résultante est agitée cinq minutes à -70°C avant l'addition, goutte à goutte, de 0,56 cm³d'iodure de méthyle. Après addition, le mélange est agité 5 minutes à -70°C puis additionné de 50 cm³ d'eau et de 7 cm³ d'une solution aqueuse d'acide phosphorique à 10 %. Le mélange obtenu revenu à 20°C est extrait par 3 fois 75 cm³ d'acétate d'éthyle. Les phases organiques rassemblées sont lavées par 75 cm³ de saumure puis séchées sur sulfate de sodium, filtrées et concentrées à sec sous pression réduite (2,7 kPa) pour donner 0,94 g d'un solide jaune que l'on purifie par HPLC préparative [Kromasil C8®, 10 µm ; éluant : eau / acétonitrile (72,5/27,5 en volumes) additionné de 0,1 % d'acide trifluoroacétique]. Après concentration des fractions contenant l'attendu sous pression réduite (2,7 kPa), on obtient une meringue blanche qui est agitée dans 20 cm³ d'éther diisopropylique puis filtrée. Le solide obtenu est séché sous pression réduite (2,7 kPa) pour donner 0,15 g de (16*R*)-16-désoxo-16-diméthylamino-20-méthyl pristinamycine II_{A}, sous forme d'une poudre blanche fondant vers 145°C.

Spectre de R.M.N. ¹H (400 MHz, CDCl₃, δ en ppm) : 0,98 (d, J = 6,5 Hz : 3H); 0,99 (d, J = 6,5 Hz : 3H) ; 1,13 (d, J = 6,5 Hz : 3H) ; 1,69 (s : 3H) ; 1,76 (mt : 1H) ; de 1,95 à 2,10 (mt: 2H) ; de 2,35 à 2,50 (mt : 1H) ; 2,35 (s : 9H) ; 2,49 (t, J = 12 Hz : 1H); de 2,60 à 2,75 (mt : 2H) ; de 2,80 à 2,90 (mt: 1H) ; 2,89 (d large, J = 12 Hz : 1H) ; 3,68 (d très large, J = 18 Hz : 1H) ; 4,07 (mt : 1H) ; 4,33 (mt : 1 H) ; 4,40 (mt : 1H) ; 4,52 (mf: 1H) ; 4,82 (d large, J = 9 Hz : 1H) ; 4,95 (dd, J = 10 et 1,5 Hz : 1H); 5,63 (ddd, J = 16 - 5 et 3,5 Hz : 1H) ; 5,88 (d large, J = 16 Hz : 1H) ; 6,05 (d large, J = 16 Hz : 1H) ; 6,12 (t, J = 3 Hz : 1H) ; 6,51 (dd, J = 16 et 8 Hz : 1H) ; 7,48 (mf: 1H).

### Exemple 4

### (16R)-20-Bromo-16-désoxo-16-fluoro pristinamycine II_{A}

En opérant comme à l'exempte 1 mais à partir de 10,6 g de (16*R*)-16-désoxo-16-fluoro pristinamycine II_{A} dans 100 cm³ de tétrahydrofuranne anhydre, additionnés en 45 minutes d'une solution de diisopropyl amidure de lithium (préalablement préparée à partir de 50 cm³ de n-butyl lithium, 300 cm³ de tétrahydrofuranne et de 11,2 cm³ de diisopropyl amine), de 12,1 cm³ de tétraméthyl éthylène diamine et de 39 g de 1,1-dibromo 1,1,2,2-tétrachloro éthane dans 100 cm³ de tétrahydrofuranne additionnés en 15 minutes. Après traitement aqueux, on obtient 43 g d'une huile brune que l'on agite durant 48 heures en présence d'éther diisopropylique. La suspension résultante est filtrée puis séchée pour donner 15 g d'un résidu orange que l'on purifie par deux chromatographies flash successives [éluant : dichlorométhane / méthanol / acétonitrile (94/3/3 en volumes) puis acétate d'éthyle / cyclohexane (70/30 en volumes)]. Après concentration sous pression réduite (2,7 kPa) des fractions contenant le produit attendu, on obtient un solide qui est agité dans 30 cm³ d'éther éthylique, filtré puis séché sous pression réduite (2,7 kPa) pour donner 1,9 g de (16*R*)-20-bromo-16-désoxo-16-fluoro pristinamycine II_{A}, sous forme d'une poudre jaune pâle fondant vers 105°C.

Spectre de R.M.N. ¹H (400 MHz, CDCl₃, δ en ppm) : 0,97 (d, J = 6,5 Hz : 3H) ; 0,99 (d, J = 6,5 Hz : 3H) ; 1,12 (d, J = 6,5 Hz : 3H) ; 1,53 (d, J = 3 Hz : 1H) ; 1,75 (s : 3H) ; de 1,90 à 2,15 (mt : 2H) ; 2,30 (dddd, J = 30 - 16 - 5 et 3 Hz : 1H) ; de 2,60 à 2,80 (mt : 2H) ; 2,83 (mt : 1H) ; 3,02 (ddd, J = 14 - 10 et 7,5 Hz : 1H) ; 3,22 (t dédoublé, J = 14 et 3 Hz : 1H) ; 3,90 (d large, J = 20 Hz : 1H) ; 4,05 (mt : 1 H) ; 4,26 (mt : 1H) ; de 4,25 à 4,40 (mf : 1H) ; 4,53 (d mf, J_{HF} = 48 Hz : 1H) ; 4,64 (mt : 1H) ; de 4,90 à 5,00 (mt : 1H) ; 4,93 (dd, J = 10 et 1,5 Hz : 1H) ; 5,69 (dt, J = 16 et 4 Hz : 1H) ; 5,90 (d large, J = 16 Hz : 1H) ; 6,01 (d large, J = 16 Hz : 1H) ; 6,19 (t, J = 3 Hz : 1H) ; 6,60 (dd, J = 16 et 7,5 Hz : 1H) ; 7,18 (mf : 1H).

### Exemple 5

### (16R)-16-Désoxo-16-fluoro-20-vinyl pristinamycine II_{B}

A une solution de 0,7 g de (16R)-16-désoxo-16-fluoro-20-iodo pristinamycine II_{B} dans un mélange de 21 cm³ de toluène et de 2,1 cm³ de N,N-diméthylformamide, on additionne rapidement, à 20°C et sous argon, 0,62 cm³ de vinyl tributyl étain puis 25 mg de tétrakis-triphénylphosphine palladium. La solution marron obtenue est chauffée 20 heures à 80°C. Le mélange réactionnel est filtré sur Clarcel^{®} et le filtrat obtenu est dilué par ajout de 50 cm³ d'acétate d'éthyle. La solution obtenue est lavée par 2 fois 30 cm³ d'eau puis par 30 cm³ de saumure. La phase organique est séchée sur sulfate de magnésium, filtrée puis concentrée sous pression réduite (2,7 kPa)pour donner un résidu solide qui est purifié par chromatographie-flash sur silice [éluant : dichlorométhane / méthanol (97/3 en volumes)]. Après concentration sous pression réduite (2,7 kPa) des fractions contenant le produit attendu, on obtient un solide qui est agité dans 30 cm³ d'éther éthylique puis filtré et séché sous pression réduite (2,7 kPa) pour donner 0,22 g de (16*R*)-16-désoxo-16-fluoro-20-vinyl pristinamycine II_{B}, sous forme d'une poudre beige fondant vers 160°C.

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,11 (d, J = 6,5 Hz : 3H) ; 1,66 (d, J = 3,5 Hz : 1H) ; de 1,70 à 2,05 (mt : 5H) ; 1,82 (s : 3H) ; 2,15 (mt : 1H) ; 2,25 (mt : 1H) ; 2,76 (mt : 1H) ; 2,92 (ddd, J = 24 - 17 et 5 Hz : 1H) ; 3,18 (t dédoublé, J = 17 et 7 Hz : 1H) ; 3,48 (mt : 1H) ; 3,73 (mt : 1H) ; 4,09 (mt : 1H) ; 4,56 (mt : 1H) ; 4,80 (dd, J = 10 et 1,5 Hz : 1H) ; de 4,80 à 4,90 (mt : 1H) ; 4,85 (dd, J = 9 et 3,5 Hz : 1H) ; 5,15 (dmt, J_{HF} = 48 Hz : 1H) ; de 5,40 à 5,50 (mt : 1H) ; 5,42 (dd, J = II et 1 Hz : 1H) ; 5,70 (ddd, J = 16 - 9,5 et 4 Hz : 1H) ; 5,83 (dd, J = 16 et 1,5 Hz : 1H) ; 5,85 (dd, J = 18 et 1 Hz : 1H) ; 5,93 (d large, J = 9,5 Hz : 1H) ; 6,24 (d large, J = 16 Hz : 1H) ; 6,52 (dd, J = 16 et 4 Hz : 1H) ; 7,13 (dd, J = 18 et 11 Hz : 1H).

La (16*R*)-16-désoxo-16-fluoro-20-iodo pristinamycine II_{B} peut être obtenue de la façon suivante.

A 2,66 g de (16*R*)-16-désoxo-16-fluoro pristinamycine II_{B} en solution dans 50 cm³ de tétrahydrofuranne anhydre, on additionne, sur une heure, à -70°C, sous atmosphère d'argon, une solution de diisopropyl amidure de lithium, refroidie à -70°C. (Cette dernière a été préalablement préparée par addition, à -40°C, de 12,4 cm³ de n-butyl lithium (1,6 M dans l'hexane) à une solution de 50 cm³ de tétrahydrofuranne et de 2,8 cm³ de diisopropyl amine suivie d'un passage à 20°C durant 5 minutes). A la solution orange résultante maintenue à -72°C, sont ajoutés, en 5 minutes, 3 cm³ de tétraméthyléthylène diamine. Après agitation du mélange réactionnel 5 minutes à -70°C, on additionne toujours à -70°C, en dix minutes, 1,27 g d'iode en solution dans 10 cm³ de tétrahydrofuranne. Le mélange est agité 15 minutes à -72°C avant addition, goutte à goutte, de 5 cm³ d'acide acétique puis de 10 cm³ d'eau une fois le mélange réactionnel revenu à 0°C. Le mélange obtenu est repris par 800 cm³ d'eau et 300 cm³ d'acétate, d'éthyle. La phase organique est séparée et la phase aqueuse extraite par 300 cm³ d'acétate d'éthyle. Les phases organiques rassemblées sont lavées par 300 cm³ d'eau additionnés d'acide chlorhydrique 1N de façon à ajuster le pH de la phase aqueuse à 2 après lavage. La phase organique résultante est séparée, successivement lavée par 50 cm³ d'eau, par une solution aqueuse de bicarbonate de sodium à 5 % et par 50 cm³ de saumure puis séchée sur sulfate de sodium, filtrée et concentrée à sec sous pression réduite (2,7 kPa) pour donner un solide marron qui est combiné avec deux autres lots obtenus dans des conditions analogues. Ce mélange (8,3 g) est purifié par chromatographie flash sur silice [éluant : dichlorométhane /méthanol (97/3 en volumes)]. Après concentration sous pression réduite (2,7 kPa), on obtient 0,75 g de (16R)-16-désoxo-16-fluoro-20-iodo pristinamycine II_{B}, sous forme d'une poudre amorphe jaune utilisée telle quelle.

Spectre de R.M.N. ¹H (400 MHz, CDCl₃, δ en ppm) : 0,94 (d, J = 6,5 Hz : 3H) ; 1,00 (d, J = 6,5 Hz : 3H) ; 1,11 (d, J = 6,5 Hz : 3H) ; de 1,55 à 2,05 (mt: 5H) ; 1,80 (s : 3H) ; . 2,15 (mt : 1H) ; 2,25 (mt : 1H) ; 2,75 (mt : 1H) ; 2,93 (ddd, J =26 - 17 et 5 Hz : 1H) ; 3,21 (t dédoublé, J = 17 et 7,5 Hz : 1H) ; 3,48 (mt : 1H) ; 3,63 (mt : 1H) ; 4,07 (mt : 1H) ; 4,56 (mt : 1H) ; 4,79 (dd, J = 10 et 1,5 Hz : 1H) ; de 4,80 à 4,90 (mt : 1H) ; 4,89 (dd, J = 9 et 4 Hz : 1H) ; 5,11 (d mt, J_{HF} = 48 Hz : 1H) ; 5,42 (d large, J = 9 Hz : 1H) ; 5,70 (ddd, J = 16 - 9 et 4 Hz : 1H) ; 5,82 (dd, J = 16 et 2 Hz : 1H) ; 5,92 (dd, J = 9 et 2,5 Hz : 1H) ; 6,24 (d large, J = 16 Hz : 1H) ; 6,52 (dd, J =16 et 5 Hz : 1H).

### Exemple 6

### 20-Méthyl pristinamycine II_{A}

A un mélange de 1,2 g de chlorure de lithium (préalablement séché sous pression réduite à 150°C), de 35 cm³ de tétrahydrofuranne anhydre et de 1,33 cm³ de diisopropylamine, on additionne, goutte à goutte, à -30°C, sous atmosphère d'argon, 5,9 cm³ de n-butyl lithium (1,6 M dans l'hexane). Après addition, le mélange est porté à 0°C avant d'être à nouveau refroidi à -70°C. Au mélange résultant sont alors ajoutés, en trente minutes, 50 cm³ d'une solution de tétrahydrofuranne anhydre contenant 1 g de pristinamycine II_{A} anhydre. La pristinamycine II_{A} anhydre a été préalablement obtenue à partir d'une solution toluène / dichlorométhane (400 cm³ /800 cm³) contenant 40 g de pristinamycine II_{A} que l'on concentre à sec, sous pression réduite.(2,7- kPa), à l'aide d'un évaporateur rotatif durant 4 heures, à 40°C maximum.

Après agitation du mélange réactionnel 5 minutes à -70°C, on additionne rapidement, 1,18 cm³ d'iodure de méthyle. Le mélange est agité 1 heure à -70°C puis réchauffé à -30°C. On ajoute alors 2 cm³ d'acide acétique; on laisse remonter la température du mélange réactionnel à 0°C puis on additionne 50 cm³ d'eau. La phase organique est séparée et la phase aqueuse extraite par 2 fois .50 cm³ d'acétate d'éthyle. Les phases organiques rassemblées sont lavées par 50 cm³ de saumure puis séchées sur sulfate de sodium, filtrées et concentrées à sec sous pression réduite (2,7 kPa) pour donner 1 g d'une huile brune que l'on purifie par cristallisation dans 15 cm³ de méthylisobutyl cétone. Le solide obtenu est filtré, lavé à l'éther diéthylique puis séché à pression atmosphérique pour donner 0,4 g de 20-méthyl pristinamycine II_{A}, sous forme de cristaux blancs fondant vers 148°C.

### Exemple 7

### (16R)-16-désoxo-16-hydroxy-20-méthyl pristinamycine II_{B}

A 15,6 cm³ de diisopropylamidure de lithium commercial (2M dans un mélange THF/heptane/éthyl benzène), on additionne, en 45 minutes, à -71°C, sous atmosphère d'argon, une solution de 3 g de (16*R*)-16-désoxo-16-hydroxy pristinamycine II_{B} anhydre et de 200 cm³ de tétrahydrofuranne anhydre. Après addition, le mélange réactionnel bleu-vert est agité dix minutes à -71°C avant addition, à -75°C, en 25 minutes, d'une solution de 0,42 cm³ d'iodure de méthyle dans 20 cm³ de tétrahydrofuranne anhydre. Après addition, le mélange est agité 1 heure à -70°C puis versé sur un mélange de 400 cm³ d'eau et de 42 cm³ d'une solution aqueuse à 10 % d'acide phosphorique. Le mélange obtenu est extrait par 3 fois 150 cm³ d'acétate d'éthyle. Les phases organiques rassemblées sont lavées par 200 cm³ de saumure, séchées sur sulfate de sodium, filtrées puis concentrée à sec sous pression réduite (2,7 kPa) pour donner un résidu que l'on purifie par HPLC préparative [Kromasil C8, 10 µm ; éluant : eau / acétonitrile (70/30 en volumes)]. Les fractions contenant le produit attendu sont concentrées sous pression réduite (2,7 kPa) pour donner une phase aqueuse qui est saturée en sel puis extraite à l'acétate d'éthyle. La phase organique est séchée sur sulfate de sodium, filtrée et concentrée à sec sous pression réduite (2,7 kPa), à 40°C, pour donner 0,27 g de (16*R*)-16-désoxo-16-hydroxy-20-méthyl pristinamycine II_{B}, sous forme d'un solide blanc fondant à partir de 131°C. La (16*R*)-16-désoxo-16-hydroxy pristinamycine II_{B} anhydre a été préalablement obtenue à partir d'une solution toluènique (300 cm³) contenant 20 g de (16*R*)-16-désoxo-16-hydroxy pristinamycine II_{B} que l'on concentre à sec, sous pression réduite (2,7 kPa), à l'aide d'un évaporateur rotatif, à 40°C maximum. Le résidu est séché sous pression réduite (2,7 kPa) pour donner 19,5 g de (16*R*)-16-désoxo-16-Hydroxy pristinamycine II_{B} anhydre.

La (16*R*)-16-désoxo-16-hydroxy pristinamycine II_{B} peut être obtenue de la façon suivante :

Une suspension de 11,35 g. de borohydrure de sodium dans 550 cm³ de dichlorométhane sont chauffés au reflux pendant 20 minutes. On ajoute alors 68,6 cm³ d'acide acétique goutte à goutte en environ 30 minutes puis une solution (préalablement séchée.sur sulfate de sodium) de 52,75 g de pristinamycine II_{B} dans 230 cm³ de dichlorométhane en environ 45 minutes. Le mélange réactionnel est agité 4,5 heures au reflux puis 16 heures à 20°C. On ajoute alors au mélange réactionnel 500 cm³ de dichlorométhane et 1500 cm³ d'eau. La phase organique est décantée et la phase aqueuse est extraite par 500 cm³ de chlorure de méthylène. Les phases organiques sont réunies et le pH est ajusté à 8 par une addition lente de 1000 cm³ d'une solution aqueuse saturée de bicarbonate de sodium. La phase organique résultante est lavée successivement par 1000 cm³ d'eau et 1000 cm³ d'une solution aqueuse saturée de chlorure de sodium puis traitée au noir 3S, séchée sur sulfate de sodium, filtrée sur Célite^{®} et concentrée à sec sous pression réduite à sec (2,7 kPa) pour donner 50 g d'un solide jaune clair. A une solution du solide précédent dans 900 cm³ de chlorure de méthylène, on ajoute à 20°C, 378 cm³ d'une solution aqueuse d'hydroxyde d'ammonium 0,5 M. Après 16 heures d'agitation à 20°C, la phase organique est décantée, lavée par 1000 cm³ d'eau puis par 1000 cm³ d'une solution aqueuse saturée de chlorure de sodium, séchée sur sulfate de sodium, filtrée et concentrée à sec sous pression réduite (2,7 kPa) pour donner 46 g d'un solide jaune pâle qui est purifié par chromatographie-flash [éluant : gradient chlorure de méthylène / méthanol (98/2 puis 97/3 en volumes)]. Après concentration des fractions, on obtient 8,57 g de (16*R*)-16-désoxo-16-hydroxy pristinamycine II_{B}, sous forme d'une meringue blanc cassé fondant vers 140°C (déc.).

Spectre de R.M.N. ¹H (400 MHz, CDCl₃, δ en ppm) : 0,96 (d, J = 6,5 Hz : 3H) ; 1,00 (d, J = 6,5 Hz : 3H) ; 1,10 (d, J = 6,5 Hz : 3H); de 1,70 à 2,05 (mt : 6H) ; 1,81 (s : 3H) ; de 2,05 à 2,20 (mt: 2H) ; 2,76 (mt : 1H); 2,84 (dd, J = 16 et 5,5 Hz : 1H); 3,00 (dd, J = 16 et 7 Hz : 1H) ; 3,04 (d, J = 4 Hz : 1H) ; 3,45 (ddd, J = 16 - 9 et 4 Hz : 1H) ; 3,90 (mt : 1H) ; 4,04 (mt : 1H) ; 4,27 (mt : 1H) ; 4,48 (ddd, J = 16 - 9 et 4Hz : 1H) ; 4,80 (dd, J = 10 et 2 Hz : 1H) ; 4,84 (dd, J = 9 et 3,5 Hz : 1H) ; 4,88 (mt : 1H) ; 5,44 (d large, J = 9 Hz : 1H) ; 5,67 (ddd, J = 16 - 9 et 4 Hz : 1H) ; 5,80 (dd, J = 16 et 1,5 Hz : 1H) ; 5,95 (dd, J = 9 et 4 Hz : 1H) ; 6,19 (d large, J = 16 Hz : 1H) ; 6,53 (dd, J = 16 et 5 Hz : 1H) ; 8,16 (s : 1H).

### Exemple 8

### (16R)-16-désoxo-16-méthoxy-20-méthyl pristinamycine II_{B}

En opérant selon les méthodes décrites dans les exemples précédents, à partir de (16*R*)-16-désoxo-16-méthoxy pristinamycine II_{B}, on prépare la (16*R*)-16-désoxo-16-méthoxy-20-méthyl pristinamycine II_{B} sous forme d'un solide jaune pâle, fondant à partir de 145°C.

Spectre de R.M.N. ¹H (300 MHz, CDCl₃, δ en ppm) : 0,97 (d, J = 6,5 Hz : 3H) ; 1,03 (d, J = 6,5 Hz : 3H) ; 1,11 (d, J = 6,5 Hz : 3H) ; de 1,70 à 2,20 (mt : 7H) ; 1,83 (s : 3H) ; 2,55 (s : 3H) ; 2,71 (dd, J = 16 et 8,5 Hz : 1H) ; 2,76 (mt : 1H) ; 3,11 (dd, J = 16 et 4 Hz : 1H) ; 3,42 (s : 3H) ; 3,55 (ddd, J = 16 - 8,5 et 3,5 Hz : 1H) ; de 3,70 à 3,90 (mt : 2H) ; 3,97 (mt : 1H) ; 4,45 (ddd, J = 16 - 8 et 4 Hz : 1H) ; de 4,65 à 4,85 (mt : 3H) ; 5,44 (d large, J = 9 Hz : 1H) ; 5,72 (ddd, J = 16 - 8,5 et 4 Hz : 1H) ; 5,82 (dd, J = 16 et 1,5 Hz : 1H) ; 5,94 (mt : 1 H) ; 6,22 (d large, J = 16 Hz : 1H) ; 6,53 (dd, J = 16 et 5 Hz : 1H).

La (16*R*)-16-désoxo-16-méthoxy-20-méthyl pristinamycine II_{B} peut être préparée comme décrit dans la demande de brevet FR 0016803.

La présente invention concerne également les compositions pharmaceutiques contenant au moins un dérivé de streptogramine selon l'invention, à l'état pur, associé à au moins un dérivé de streptogramine du groupe B, le cas échéant sous forme de sel, et/ou sous forme d'une association avec un ou plusieurs diluants ou adjuvants compatibles et pharmaceutiquement acceptables.

Les compositions selon l'invention peuvent être utilisées par voie orale, parentérale, topique, rectale ou en aérosols.

Comme compositions solides pour administration orale peuvent être utilisés des comprimés, des pilules, des gélules, des poudres ou des granulés. Dans ces compositions, le produit actif selon l'invention, généralement sous forme d'association est mélangé à un ou plusieurs diluants ou adjuvants inertes, tels que saccharose, lactose ou amidon. Ces compositions peuvent comprendre des substances autres que les diluants, par exemple un lubrifiant tel que le stéarate de magnésium ou un enrobage destiné à une, libération contrôlée.

Comme compositions liquides pour administration orale, on peut utiliser des solutions pharmaceutiquement acceptables, des suspensions, des émulsions, des sirops et des élixirs contenant des diluants inertes tels que l'eau ou l'huile de, paraffine. Ces compositions peuvent également comprendre des substances autres que les diluants, par exemple des produits mouillants, édulcorants ou aromatisants.

Les compositions pour administration parentérale, peuvent être des solutions stériles ou des émulsions. Comme solvant ou véhicule, on peut employer le propylèneglycol, un polyéthylèneglycol, des huiles végétales, en particulier l'huile d'olive, des esters organiques injectables, par exemple l'oléate d'éthyle. Ces compositions peuvent également contenir des adjuvants, en particulier des agents mouillants, isotonisants, émulsifiants, dispersants et stabilisants.

La stérilisation peut se faire de plusieurs façons, par exemple à l'aide d'un filtre bactériologique, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration topique peuvent être par exemple des crèmes, des pommades, des lotions ou des aérosols.

Les compositions par administration rectale sont les suppositoires ou les capsules rectales, qui contiennent outre le principe actif, des excipients tels que le beurre de cacao, des glycérides semi-synthétiques ou des polyéthylèneglycols.

Les compositions peuvent également être des aérosols. Pour l'usage sous forme d'aérosols liquides, les compositions peuvent être des solutions stériles stables ou des compositions solides dissoutes au moment de l'emploi dans de l'eau stérile apyrogène, dans du sérum ou tout autre véhicule pharmaceutiquement acceptable. Pour l'usage sous forme d'aérosols secs destinés à être directement inhalés, le principe actif est finement divisé et associé à un diluant ou véhicule solide hydrosoluble d'une granulométrie de 30 à 80 µm, par exemple le dextrane, le mannitol ou le lactose.

En thérapeutique humaine, les nouveaux dérivés de streptogramine selon l'invention sont particulièrement utiles dans le traitement des infections d'origine bactérienne. Les doses dépendent de l'effet recherché et de la durée du traitement. Le médecin déterminera la posologie qu'il estime la plus appropriée en fonction du traitement, en fonction de l'âge, du poids, du degré de l'infection et des autres facteurs propres au sujet à traiter. Généralement, les doses sont comprises entre 0,5 et 3 g de produit actif en 2 ou 3 administrations par jour, par voie orale ou parentérale pour un adulte.

L'exemple suivant illustre une composition selon l'invention.

### EXEMPLE

On prépare selon la technique habituelle des comprimés dosés à 250 mg de produit actif, ayant la composition suivante :
- (16*R*)-20-bromo-16-désoxo-16-fluoro pristinamycine II_{A} 175 mg
- pristinamycine I_{B} 75 mg
- excipient : amidon, silice hydratée, dextrine, gélatine, stéarate de magnésium : qsp 500 mg

## Revendications

1. Un dérivé du groupe A des streptogramines de formule générale : 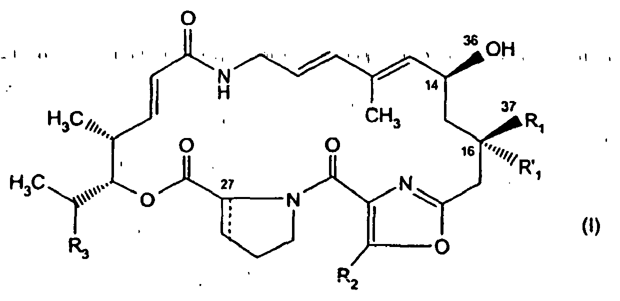 dans laquelle :
■ R₁ représente un atome d'halogène ou un radical hydroxy, alcoyloxy, azido, thiocyanato, ou R₁ représente un radical -NR'R" pour lequel R' est un atome d'hydrogène ou un radical méthyle, et R" est un atome d'hydrogène, un radical alcoyle, cycloalcoyle, allyle, propynyle, benzyle, ou -OR"', R"' étant un atome d'hydrogène, un radical alcoyle, cycloalcoyle, allyle, propynyle ou benzyle, ou bien R" représente -NR°R°°, R° et R°° pouvant représenter un radical méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, et
■ R'₁ représente un atome d'hydrogène ou bien,
■ R₁ et R'₁ forment ensemble un radical oxo,
■ R₂ représente un atome d'halogène ou un radical alcoyle, alcènyle ou alcynyle, phényle ou hétéroaryle, ou un radical alcoylthio, phénylthio ou hétéroarylthio,
■ R₃ est un atome d'hydrogène ou un radical méthyle ou éthyle, et
■ la liaison 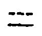 représente une liaison simple (stéréochimie 27R) ou une liaison double,
les radicaux alcoyle contenant 1 à 6 carbones en chaîne droite ou ramifiée et les radicaux alcényle et/ou alcynyle contenant 2 à 6 carbones en chaîne droite ou ramifiée,
ainsi que ses sels lorsqu'ils existent.

2. Un procédé de préparation d'un dérivé du groupe A des streptogramines selon la revendication 1, **caractérisé en ce que** l'on effectue la métallation d'un dérivé de streptogramine de formule générale : 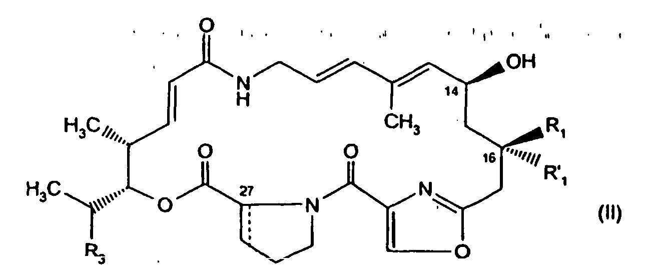 dans laquelle R'₁, R₃ et la liaison 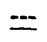 sont définis comme précédemment et R₁ représente un atome de fluor ou un radical hydroxy, alcoyloxy, ou représente un radical -NR'R" pour lequel R' est H ou un radical méthyle, et R" est H, un radical alcoyle, cycloalcoyle, allyle, propynyle, benzyle, ou -OR"', R"' étant H, alcoyle, cycloalcoyle, allyle, propynyle ou benzyle, ou bien R" représente -NR°R°°, R° et R°° pouvant être méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre, ou bien, R₁ et R'₁ forment ensemble un radical oxo, par action successivement d'un amidure, puis par action d'un agent électrophile pouvant être représenté par la formule générale :
R₂-X (III)
dans laquelle R₂ est un atome d'halogène, un radical alcoyle, alcènyle ou alcynyle, à l'exception de représenter un radical vinyle ou éthynyle, et X représente un atome d'halogène ou un radical sulfonyloxy, ou bien par action d'un disulfure lorsque R₂ est alcoylthio, phénylthio ou hétéroarylthio, suivie le cas échéant de la transformation d'un dérivé de streptogramine selon la revendication 1, pour lequel R₂ est un atome de chlore, brome ou iode, en un dérivé de streptogramine selon la revendication 1, pour lequel R₂ est radical vinyle, éthynyle, phényle ou hétéroaryle et/ou le cas échéant, de la transformation du dérivé de streptogramine selon la revendication 1, pour lequel R₁ et R'₁ forment ensemble un radical oxo en un dérivé correspondant pour lequel R₁ est un atome de chlore, de brome ou d'iode ou un radical azido ou thiocyanato et R'₁ est un atome d'hydrogène, et/ou suivie de la transformation d'un dérivé de streptogramine selon la revendication 1 pour lequel la liaison 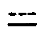 est une liaison double en un dérivé de streptogramine selon la revendication 1 pour lequel la liaison 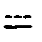 est une liaison simple.

3. Un procédé de préparation selon la revendication 2, d'un dérivé du groupe A des streptogramines selon la revendication 1, **caractérisé en ce que** l'agent électrophile peut être un donneur d'halogène choisi parmi le N-bromo ou N-chloro succinimide, le 1,1,2,2-tétrafluoro-1,2-dichloro ou dibromo éthane, le 1,2-dibromo-1,1,2,2-tétrachloro éthane, le 1,2-diiodoéthane ou le 1,2-dibromoéthane, le 1-chloro-2-iodo éthane ou l'hexachloroacétone.

4. Un polyanion dérivé du groupe A des streptogramines répondant, selon la nature des substituants et selon que la liaison 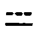 est une liaison double ou une liaison simple, aux structures de formules générales : 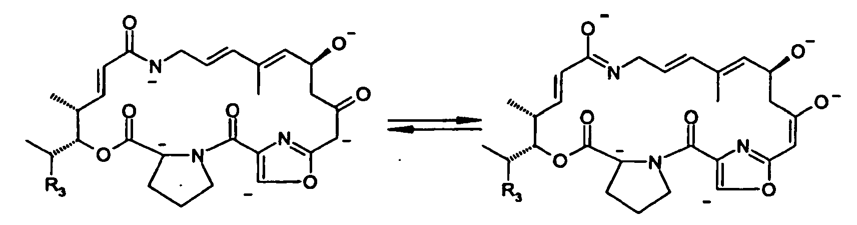 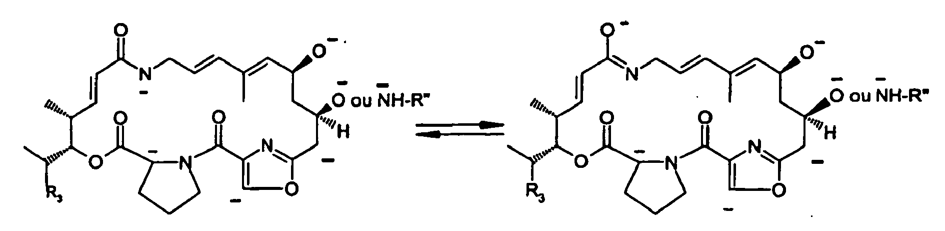 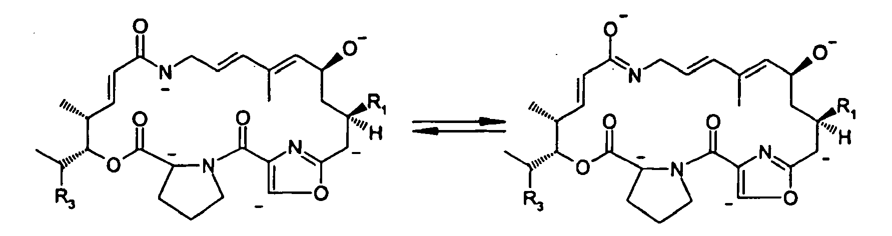 ou 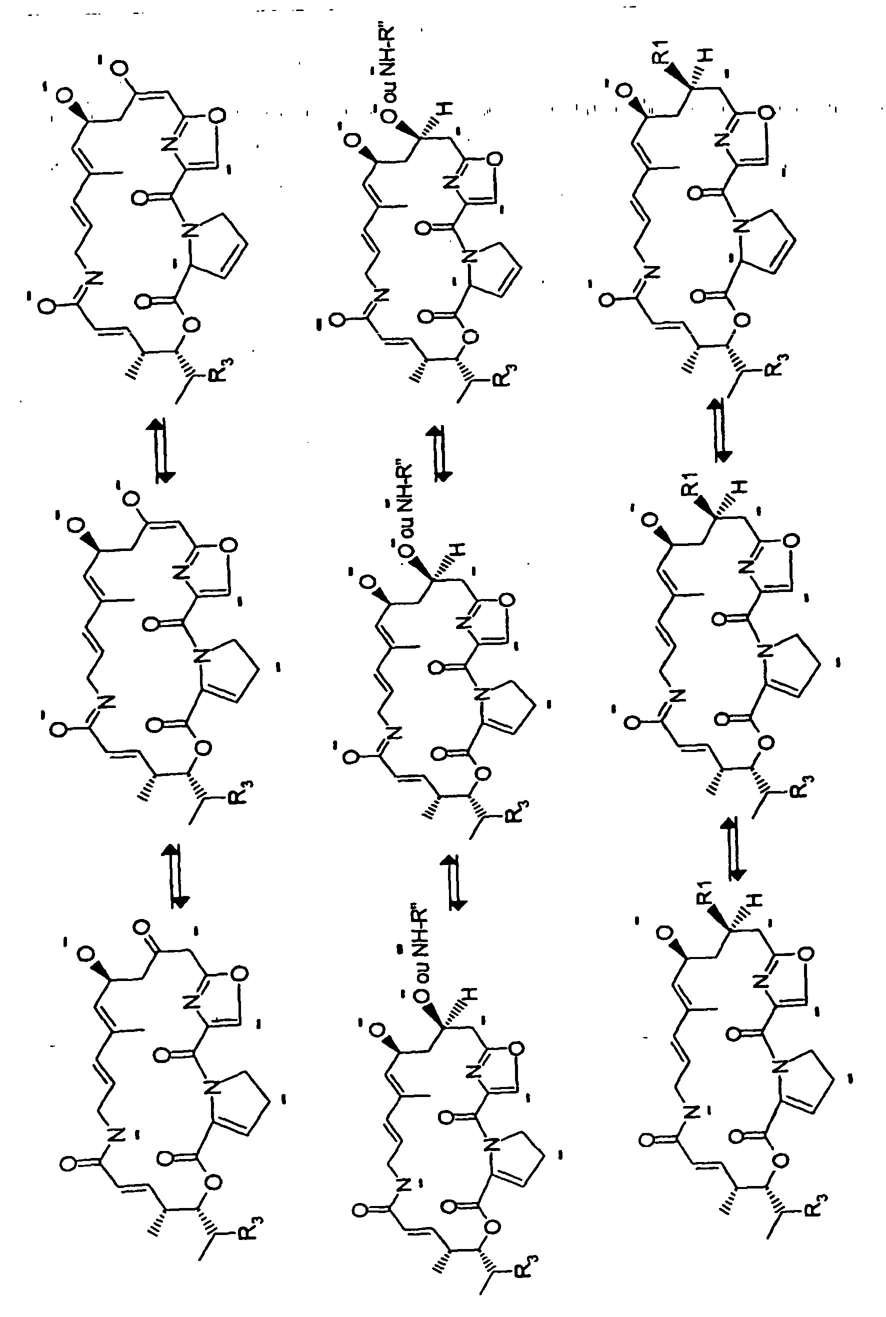 et pour lesquelles R3 est défini comme dans la revendication 1, et R, représente un atome de fluor ou un radical alcoyloxy, ou représente un radical -NR'R" pour lequel R' est un radical méthyle, et R" est un radical alcoyle, cycloalcoyle, allyle, propynyle, benzyle, ou -OR"', R'" étant H, alcoyle, cycloalcoyle, allyle, propynyle ou benzyle, ou bien R" représente-NR° R°° ,R° et R°° pouvant être méthyle, ou former ensemble avec l'atome d'azote auquel ils sont attachés un hétérocycle à 4 ou 5 chaînons saturé ou insaturé pouvant en outre contenir un autre hétéroatome choisi parmi l'azote, l'oxygène ou le soufre.

5. Composition pharmaceutique comprenant un dérivé de streptogramine du groupe A selon la revendication 1, à l'état pur ou sous forme d'association avec au moins un dérivé de streptogramine du groupe B et/ ou sous forme d'association avec un ou plusieurs diluants ou adjuvants compatibles et pharmaceutiquement acceptables.

6. Un dérivé du groupe des streptogramines A selon la revendication 1, **caractérisé en ce que** R2 est un radical hétéroaryle ou hétéroarylthio, ce dernier étant mono ou bicyclique et comprenant 1 à 4 hétéroatomes choisis parmi l'azote, l'oxygène ou le soufre, ainsi que ses sels lorsqu'ils existent.

7. Un dérivé du groupe des streptogramines A selon la revendication 6, **caractérisé en ce que** le radical hétéroaryle peut être choisi parmi pyridyle, thiényle, furyle, pyrrolyle, imidazolyle, pirazinyle, quinolyle, isoquinolyle, indolyle, oxazolyle, thiazolyle, pyrazolyle, indazolyle, thiadiazolyle ou oxadiazolyle, ainsi que ses sels lorsqu'ils existent.

8. Un dérivé du groupe des streptogramines A selon la revendication 1, choisi parmi
- (16R)-20-Bromo-16-désoxo-16-fluoro pristinamycine II_{B}
- (16R)-16-Désoxo-16-fluoro-20-méthyl-pristinamycine II_{B}
- (16R)-16-Désoxo-16-diméthylamino-20-méthyl-pristinamycine II_{B}
- (16R)-20-Bromo-16-désoxo-16-fluoro pristinamycine II_{A}
- (16R)-16-Désoxo-16-fluoro-20-vinyl pristinamycine II_{B}
- 20-Méthyl pristinamycine II
- (16R)-16-désoxo-16-hydroxy-20-méthyl pristinamycine II_{B} et
- (16R)-16-désoxo-16-méthoxy-20-méthyl pristinamycine II_{B}
ainsi que ses sels lorsqu'ils existent.

## Claims

1. Group A streptogramin derivative of general formula: 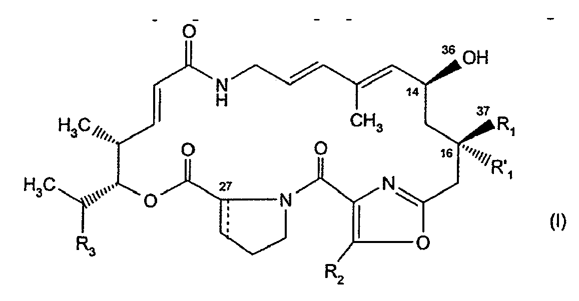 in which:
• R₁ represents a halogen atom or a hydroxyl, alkyloxy, azido or thiocyanato radical, or R₁ represents a radical -NR'R" for which R' is a hydrogen atom or a methyl radical and R" is a hydrogen atom or an alkyl, cycloalkyl, allyl, propynyl or benzyl radical, or -OR"', R"' being a hydrogen atom or an alkyl, cycloalkyl, allyl, propynyl or benzyl radical, or alternatively R" represents -NR°R°°, R° and R°° possibly representing a methyl radical, or forming, together with the nitrogen atom to which they are attached, a saturated or unsaturated 4- or 5-membered heterocycle also possibly containing another hetero atom chosen from nitrogen, oxygen and sulfur, and
• R'₁ represents a hydrogen atom, or
• R₁ and R'₁ together form an oxo radical,
• R₂ represents a halogen atom or an alkyl, alkenyl or alkynyl, phenyl or heteroaryl radical, or an alkylthio, phenylthio or heteroarylthio radical,
• R₃ is a hydrogen atom or a methyl or ethyl radical, and
• the bond 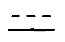 represents a single bond (27R stereochemistry) or a double bond,
the alkyl radicals containing 1 to 6 carbons in a straight or branched chain and the alkenyl and/or alkynyl radicals containing 2 to 6 carbons in a straight or branched chain,
and also the salts thereof, when they exist.

2. Process for preparing a group A streptogramin derivative according to Claim 1, **characterized in that** metalation is carried out on a streptogramin derivative of general formula: 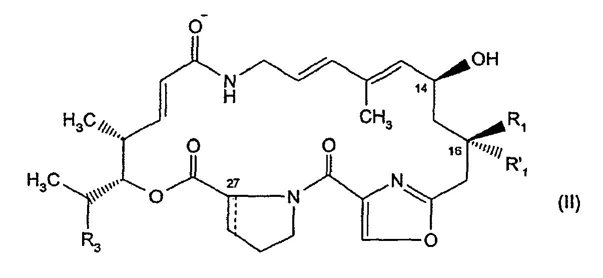 in which R'₁, R₃ and the bond 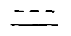 are defined as above and R₁ represents a fluorine atom or a hydroxyl or alkyloxy radical, or represents a radical -NR'R" for which R' is H or a methyl radical and R" is H or an alkyl, cycloalkyl, allyl, propynyl or benzyl radical, or -OR"', R"' being H, alkyl, cycloalkyl, allyl, propynyl or benzyl, or alternatively R" represents -NR°R°°, R° and R°° possibly being methyl, or forming, together with the nitrogen atom to which they are attached, a saturated or unsaturated 4- or 5-membered heterocycle also possibly containing another hetero atom chosen from nitrogen, oxygen and sulfur, or alternatively R₁ and R'₁ together form an oxo radical, by the action successively of an amide and then by the action of an electrophilic agent which may be represented by the general formula:
R₂-X (III)
in which R₂ is a halogen atom, an alkyl, alkenyl or alkynyl radical, with the exception of representing a vinyl or ethynyl radical, and X represents a halogen atom or a sulfonyloxy radical, or alternatively by the action of a disulfide when R₂ is alkylthio, phenylthio or heteroarylthio, followed, where appropriate, by the conversion of a streptogramin derivative according to Claim 1, for which R₂ is a chlorine, bromine or iodine atom, into a streptogramin derivative according to Claim 1, for which R₂ is vinyl, ethynyl, phenyl or heteroaryl radical and/or, where appropriate, by the sconversion of the streptogramin derivative according to Claim 1, for which R₁ and R'₁ together form an oxo radical, into a corresponding derivative for which R₁ is a chlorine, bromine or iodine atom or an azido or thiocyanato radical and R'₁ is a hydrogen atom, and/or followed by the conversion of a streptogramin derivative according to Claim 1, for which the bond 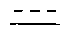 is a double bond, into a streptogramin derivative according to Claim 1, for which the bond 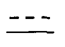 is a single bond.

3. Process, according to Claim 2, for preparing a group A streptogramin derivative according to Claim 1, **characterized in that** the electrophilic agent may be a halogen donor chosen from N-bromosuccinimide or N-chlorosuccinimide, 1,1,2,2-tetrafluoro-1,2-dichloro- or - dibromoethane, 1,2-dibromo-1,1,2,2-tetrachloroethane, 1,2-diiodoethane or 1,2-dibromoethane, 1-chloro-2-iodoethane and hexachloroacetone.

4. Polyanion derived from group A streptogramins corresponding, depending on the nature of the substituents and depending on whether the bond 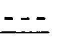 is a double bond or a single bond, to the structures of general formulae: 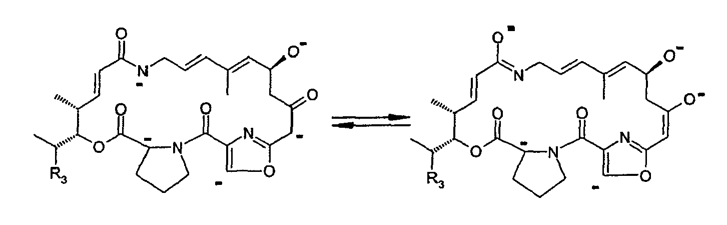 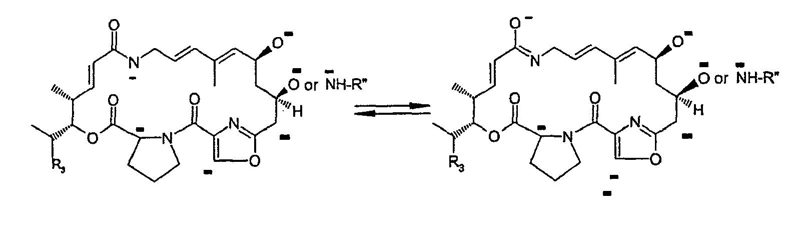 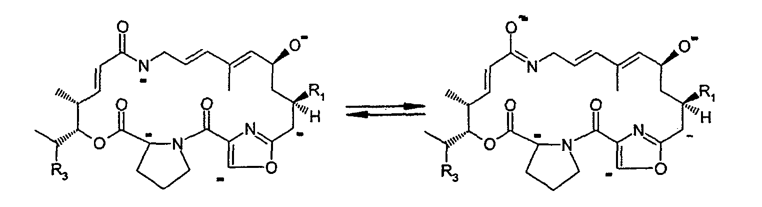 or 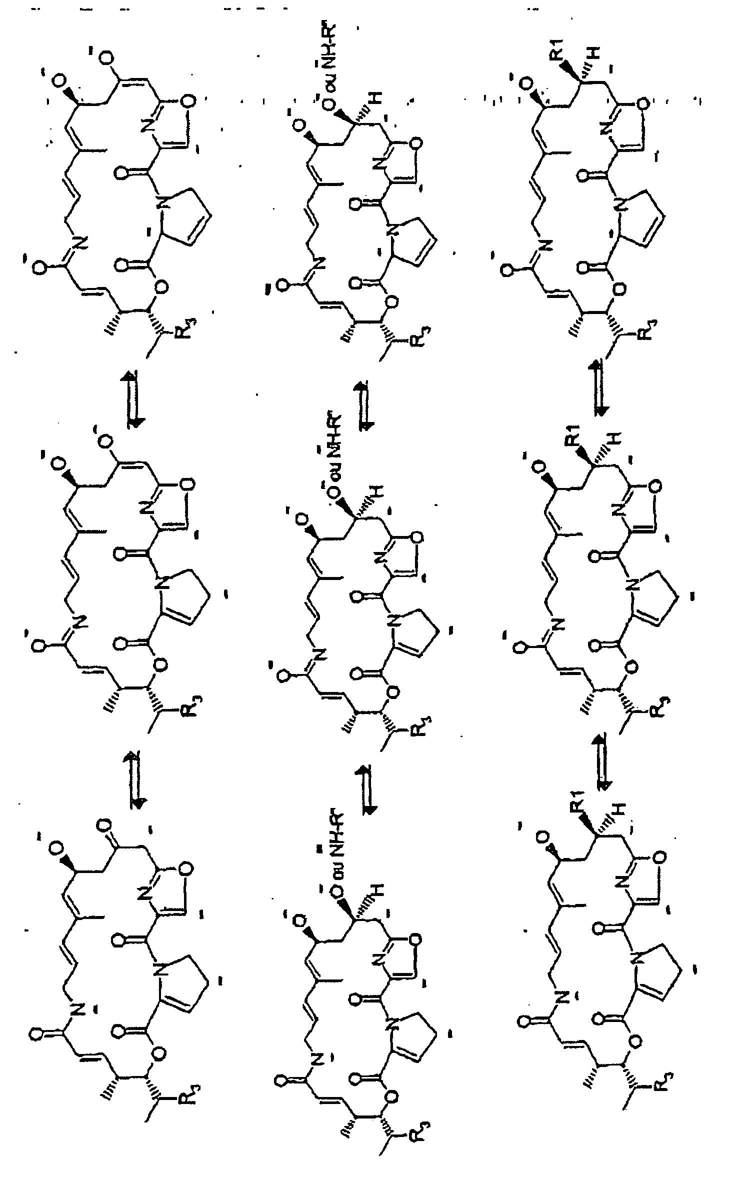 and for which R₃ is defined as in Claim 1, and R₁ represents a fluorine atom or an alkyloxy radical, or represents a radical -NR'R" for which R' is a methyl radical and R" is an alkyl, cycloalkyl, allyl, propynyl or benzyl radical, or -OR"', R"' being H, alkyl, cycloalkyl, allyl, propynyl or benzyl, or alternatively R" represents -NR°R°°, R° and R°° possibly being methyl, or forming, together with the nitrogen atom to which they are attached, a saturated or unsaturated 4- or 5-membered heterocycle also possibly containing another hetero atom chosen from nitrogen, oxygen and sulfur.

5. Pharmaceutical composition comprising a group A streptogramin derivative according to Claim 1, in pure form or in the form of a combination with at least one group B streptogramin derivative, and/or in the form of a combination with one or more compatible and pharmaceutically acceptable diluents or adjuvants.

6. Derivative of the streptogramin A group according to Claim 1, **characterized in that** R₂ is a heteroaryl or heteroarylthio radical, this radical being monocyclic or bicyclic and comprising 1 to 4 heteroatoms chosen from nitrogen, oxygen and sulfur, and also the salts thereof, when they exist.

7. Derivative of the streptogramin A group according to Claim 6, **characterized in that** the heteroaryl radical may be chosen from pyridyl, thienyl, furoyl, pyrrolyl, imidazolyl, pyrazinyl, quinolyl, isoquinolyl, indolyl, oxazolyl, thiazolyl, pyrazolyl, indazolyl, thiadiazolyl and oxadiazolyl, and also the salts thereof, when they exist.

8. Derivative of the streptogramin A group according to Claim 1, chosen from:
- (16R)-20-bromo-16-deoxo-16-fluoropristinamycin II_{B}
- (16R)-20-deoxo-16-fluoro-20-methylpristinamycin II_{B}
- (16R)-16-deoxo-16-dimethylamino-20-methylpristinamycin II_{B}
- (16R)-20-bromo-16-deoxo-16-fluoropristinamycin II_{A}
- (16R)-16-deoxo-16-fluoro-20-vinylpristinamycin II_{B}
- 20-methylpristinamycin II
- (16R)-16-deoxo-16-hydroxy-20-methylpristinamycin II_{B} and
- (16R)-16-deoxo-16-methoxy-20-methylpristinamycin II_{B}
and the salts thereof, when they exist.

## Patentansprüche

1. Gruppe-A-Streptogramin-Derivat der allgemeinen Formel: 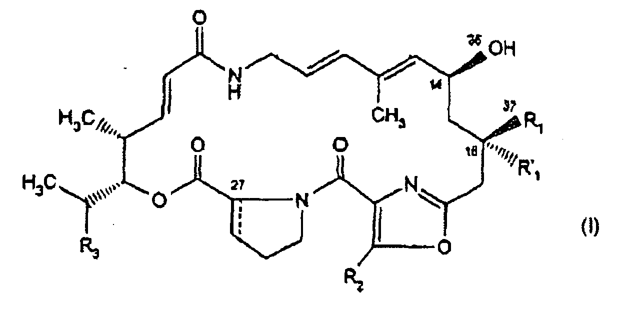 worin:
■ R₁ für ein Halogenatom oder einen Hydroxy-, Alkyloxy-, Azido- oder Thiocyanatorest oder einen Rest -NR'R'', worin R' ein Wasserstoffatom oder einen Methylrest bedeutet und R" ein Wasserstoffatom, einen Alkyl-, Cycloalkyl-, Allyl-, Propinyl- oder Benzylrest oder -OR"', worin R"' für ein Wasserstoffatom oder einen Alkyl-, Cycloalkyl-, Allyl-, Propinyl- oder Benzylrest steht, oder auch - NR°R°°, worin R° und R°° für einen Methylrest stehen können oder gemeinsam mit dem Stickstoffatom, an das sie gebunden sind, einen gesättigten oder ungesättigten 4- oder 5-gliedrigen Heterocyclus, der außerdem noch ein anderes, unter Stickstoff, Sauerstoff und Schwefel ausgewähltes Heteroatom enthalten kann, bilden können, bedeutet, und
■ R'₁ für ein Wasserstoffatom steht oder
■ R₁ und R'₁ gemeinsam einen Oxorest bilden,
■ R₂ für ein Halogenatom oder einen Alkyl-, Alkenyl-, Alkinyl-, Phenyl-, Heteroaryl-, Alkylthio-, Phenylthio- oder Heteroarylthiorest steht,
■ R₃ für ein Wwasserstoffatom oder einen Methyl-oder Ethylrest steht und
■ die Bindung 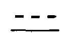 für eine Einfachbindung (27R-Stereochemie) oder eine Doppelbindung steht,
wobei die Alkylreste 1 bis 6 Kohlenstoffatome in gerader oder verzweigter Kette enthalten und die Alkenyl- und/oder Alkinylreste 2 bis 6 Kohlenstoffatome in gerader oder verzweigter Kette enthalten,
sowie Salze davon, sofern sie existieren.

2. Verfahren zur Herstellung eines Gruppe-A-Streptogramin-Derivats gemäß Anspruch 1, **gekennzeichnet durch** Metallierung eines Streptogramin-Derivats der allgemeinen Formel: 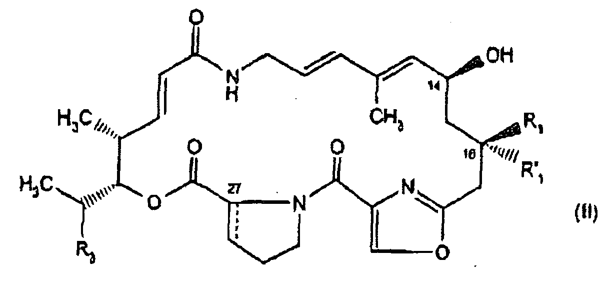 worin R'₁, R₃ und die Bindung 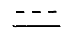 die oben angegebene Bedeutung besitzen und R₁ für ein Fluoratom oder einen Hydroxy- oder Alkyloxyrest oder einen Rest -NR'R" worin R' ein Wasserstoffatom oder einen Methylrest bedeutet und R" ein Wasserstoffatom, einen Alkyl-, Cycloalkyl-, Allyl-, Propinyl- oder Benzylrest oder -OR''', worin R"' für ein Wasserstoffatom oder einen Alkyl-, Cycloalkyl-, Allyl-, Propinyl- oder Benzylrest steht, oder auch -NR°R°°, worin R° und R°° für Methyl stehen können oder gemeinsam mit dem Stickstoffatom, an das sie gebunden sind, einen gesättigten oder ungesättigten 4- oder 5-gliedrigen Heterocyclus, der außerdem noch ein anderes, unter Stickstoff, Sauerstoff und Schwefel ausgewähltes Heteroatom enthalten kann, bilden können, bedeutet oder R₁ und R'₁ gemeinsam einen Oxorest bilden,
**durch** aufeinanderfolgende Einwirkung eines Amids und dann **durch** Einwirkung eines elektrophilen Agens, das **durch** die folgende allgemeine Formel wiedergegeben werden kann:
R₂-X (III)
worin R₂ für ein Halogenatom oder einen Alkyl-, Alkenyl- oder Alkinylrest mit Ausnahme eines Vinyl- oder Ethinylrests steht und X für ein Halogenatom oder einen Sulfonyloxyrest steht, oder auch **durch** Einwirkung eines Disulfids für den Fall, daß R₂ für Alkylthio, Phenylthio oder Heteroarylthio steht, gegebenenfalls gefolgt von der Umwandlung eines Streptogramin-Derivats nach Anspruch 1, worin R₂ für ein Chlor-, Brom- oder Iodatom steht, in ein Streptogramin-Derivat nach Anspruch 1, worin R₂ für einen Vinyl-, Ethinyl-, Phenyl- oder Heteroarylrest steht, und/oder gegebenenfalls der Umwandlung des Streptogramin-Derivats nach Anspruch 1, worin R₁ und R'₁ gemeinsam einen Oxorest bilden, in ein entsprechendes Derivat, in dem R₁ für ein Chlor-, Brom- oder Iodatom oder einen Azido- oder Thiocyanatorest steht und R'₁ für ein Wasserstoffatom steht, und/oder gefolgt von der Umwandlung eines Streptogramin-Derivats nach Anspruch 1, worin die Bindung 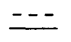 für eine Doppelbindung steht, in ein Streptogramin-Derivats nach Anspruch 1, worin die Bindung 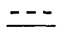 für eine Einfachbindung steht.

3. Herstellungsverfahren nach Anspruch 2 zur Herstellung eines Gruppe-A-Streptogramin-Derivats gemäß Anspruch 1, **dadurch gekennzeichnet, daß** es sich bei dem elektrophilen Agens um einen unter N-Brom- oder N-Chlorsuccinimid, 1,1,2,2-Tetrafluor-1,2-dichlorethan oder -dibromethan, 1,2-Dibrom-1,1,2,2-tetrachlorethan, 1,2-Diiodethan, 1,2-Dibromethan, 1-Chlor-2-iodethan oder Hexachloraceton ausgewählten Halogen-Donor handeln kann.

4. Von Gruppe-A-Streptograminen abgeleitetes Polyanion, das je nach Art der Substituenten und je nachdem, ob die Bindung 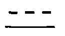 für eine Doppelbindung oder eine Einfachbindung steht, den Strukturen der folgenden allgemeinen Formeln entspricht: 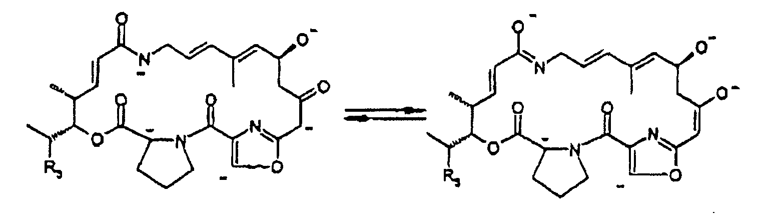 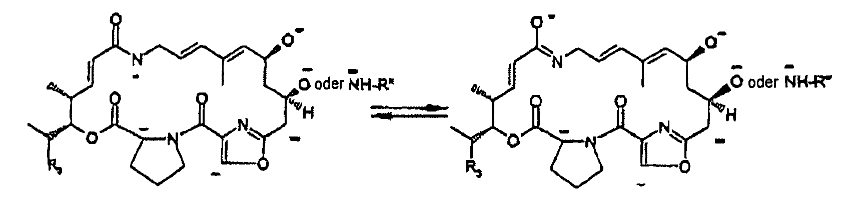 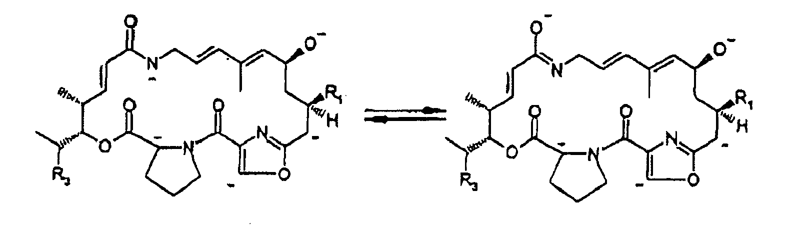 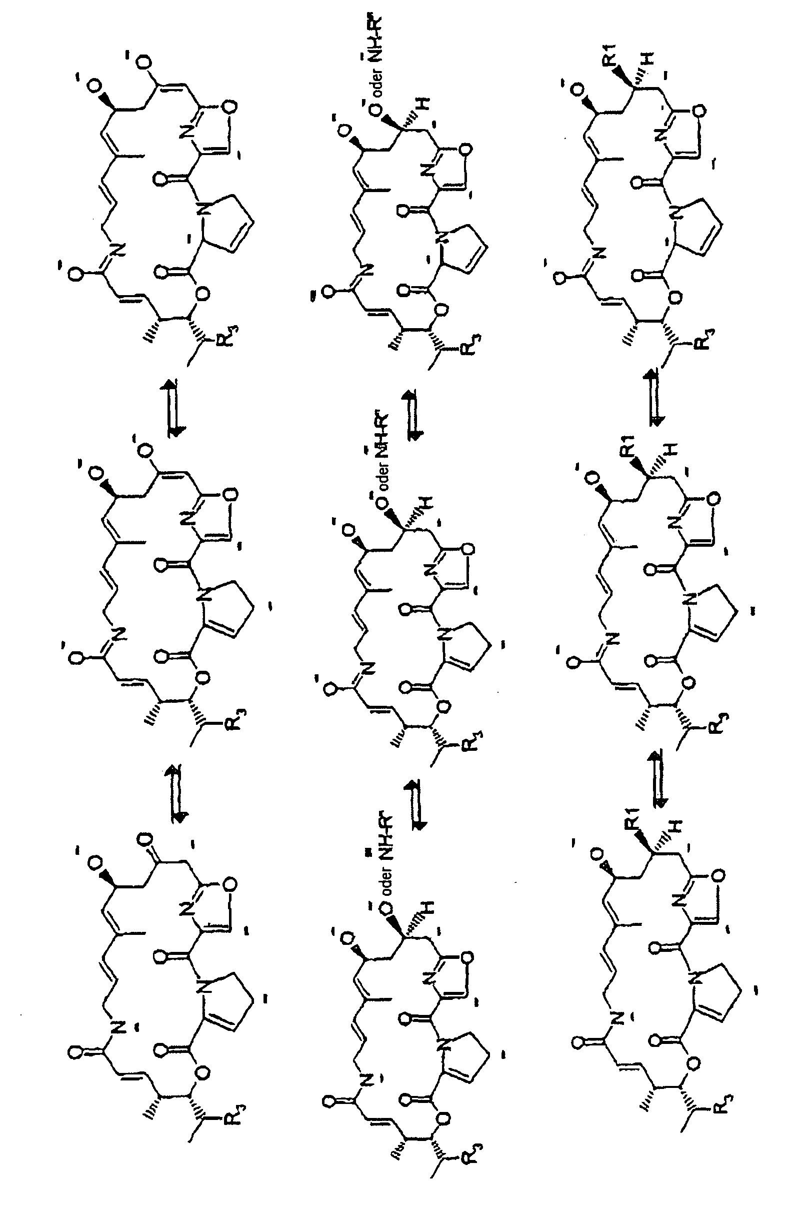 worin R3 die in Anspruch 1 angegebene Bedeutung besitzt und R für ein Fluoratom, einen Alkyloxyrest oder einen Rest NR'R", worin R' ein einen Methylrest bedeutet und R" einen Alkyl-, Cycloalkyl-, Allyl-, Propinyl- oder Benzylrest oder -OR"', worin R"' für ein Wasserstoffatom oder einen Alkyl-, Cycloalkyl-, Allyl-, Propinyl-oder Benzylrest steht, oder auch -NR°R°°, worin R° und R°° für einen Methylrest stehen können oder gemeinsam mit dem Stickstoffatom, an das sie gebunden sind, einen gesättigten oder ungesättigten 4- oder 5-gliedrigen Heterocyclus, der außerdem noch ein anderes, unter Stickstoff, Sauerstoff und Schwefel ausgewähltes Heteroatom enthalten kann, bilden können, bedeutet.

5. Pharmazeutische Zusammensetzung, enthaltend ein Gruppe-A-Streptogramin-Derivat gemäß Anspruch 1 in reiner Form oder in Form einer Kombination mit mindestens einem Gruppe-B-Streptogramin-Derivat und/oder in Form einer Kombination mit einem oder mehreren verträglichen und pharmazeutisch unbedenklichen Verdünnungsmitteln oder Hilfsstoffen.

6. Gruppe-A-Streptogramin-Derivat gemäß Anspruch 1, **dadurch gekennzeichnet, daß** R2 für einen Heteroaryl- oder Heteroarylthiorest steht, wobei letzterer mono- oder bicyclisch sein kann und 1 bis 4 unter Stickstoff, Sauerstoff oder Schwefel ausgewählte Heteroatome enthält, sowie Salze davon, sofern sie existieren.

7. Gruppe-A-Streptogramin-Derivat gemäß Anspruch 6, **dadurch gekennzeichnet, daß** der Heteroarylrest unter Pyridyl, Thienyl, Furyl, Pyrrolyl, Imidazolyl, Pirazinyl, Chinolyl, Isochinolyl, Indolyl, Oxazolyl, Thiazolyl, Pyrazolyl, Indazolyl, Thiadiazolyl oder Oxadiazolyl ausgewählt sein kann, sowie Salze davon, sofern sie existieren.

8. Gruppe-A-Streptogramin-Derivat gemäß Anspruch 1, ausgewählt unter
- (16R)-20-Brom-16-deoxo-16-fluorpristinamycin II_{B}
- (16R)-16-Deoxo-16-fluor-20-methylpristinamycin II_{B}
- (16R)-16-Deoxo-16-dimethylamino-20-methylpristinamycin II_{B}
- (16R)-20-Brom-16-deoxo-16-fluorpristinamycin II_{A}
- (16R)-16-Deoxo-16-fluor-20-vinylpristinamycin II_{B}
- 20-Methylpristinamycin II_{B}
- (16R)-16-Deoxo-16-hydroxy-20-methylpristinamycin II_{B} und
- (16R)-16-Deoxo-16-methoxy-20-methylpristinamycin II_{B}
sowie Salzen davon, sofern sie existieren.
